# EUROPÄISCHE PATENTSCHRIFT

(11) **EP 1 611 076 B1**
(45) Veröffentlichungstag und Bekanntmachung des Hinweises auf die Patenterteilung: **19.03.2008**
(21) Anmeldenummer: 04721853.2
(22) Anmeldetag: 19.03.2004
(51) Int. Cl.: C07C 57/05

(54) **VERFAHREN DER HETEROGEN KATALYSIERTEN PARTIELLEN GASPHASENOXIDATION VON PROPEN ZU ACRYLSÄURE**
METHOD FOR THE HETEROGENICALLY CATALYSED PARTIAL GAS PHASE OXIDATION OF PROPENE TO FORM ACRYLIC ACID
PROCEDE DE L'OXYDATION EN PHASE GAZEUSE PARTIELLE CATALYSEE HETEROGENE DE PROPENE POUR OBTENIR UN ACIDE ACRYLIQUE

(30) Priorität: 25.03.2003 DE 10313208; 05.06.2003 US 475780 P
(43) Veröffentlichungstag der Anmeldung: 04.01.2006
(73) Patentinhaber: BASF SE, 67056 Ludwigshafen (DE)
(72) Erfinder: DIETERLE, Martin, 68167 Mannheim (DE); PETZOLDT, Jochen, 68163 Mannheim (DE); MÜLLER-ENGEL, Klaus, Joachim, 76297 Stutensee (DE); ARNOLD, Heiko, Zijin Villas, Nanjing 210014 (CN)
(86) Internationale Anmeldenummer: PCT/EP2004/002879
(87) Internationale Veröffentlichungsnummer: WO 2004/085367

(56) Entgegenhaltungen:
- DE-A- 19 955 168

## Beschreibung

Vorliegende Erfindung betrifft ein Verfahren der heterogen katalysierten partiellen Gasphasenoxidation von Propen zu Acrylsäure, bei dem man ein Propen, molekularen Sauerstoff und wenigstens ein Inertgas enthaltendes Reaktionsgasausgangsgemisch 1, das den molekularen Sauerstoff und das Propen in einem molaren Verhältnis O₂ : C₃H₆ ≥1 enthält, in einer ersten Reaktionsstufe so über eine Festbettkatalysatorschüttung 1, die in zwei räumlich aufeinanderfolgenden Reaktionszonen A, B angeordnet ist, wobei die Temperatur der Reaktionszone A eine Temperatur im Bereich von 290 bis 380°C und die Temperatur der Reaktionszone B ebenfalls eine Temperatur im Bereich von 290 bis 380°C ist, und deren Aktivmasse wenigstens ein die Elemente Mo, Fe und Bi enthaltendes Multimetalloxid ist, führt, dass sich die Reaktionszone A bis zu einem Umsatz des Propens von 40 bis 80 mol.-% erstreckt und der Propenumsatz bei einmaligem Durchgang durch die Festbettkatalysatorschüttung 1 ≥90 mol.-% und die damit einhergehende Selektivität der Acroleinbildung sowie der Acrylsäurenebenproduktbildung zusammengenommen ≥90 mol.-% betragen, die Temperatur des die erste Reaktionsstufe verlassenden Produktgasgemisches durch Kühlung gegebenenfalls verringert und dem Produktgasgemisch gegebenenfalls molekularen Sauerstoff und/oder Inertgas zugibt, und danach das Produktgasgemisch als Acrolein, molekularen Sauerstoff und wenigstens ein Inertgas enthaltendes Reaktionsgasausgangsgemisch 2, das den molekularen Sauerstoff und das Acrolein in einem molaren Verhältnis O₂ : C₃H₄O ≥ 0,5 enthält, in einer zweiten Reaktionsstufe so über eine Festbettkatalysatorschüttung 2, die in zwei räumlich aufeinanderfolgenden Reaktionszonen C, D angeordnet ist, wobei die Temperatur der Reaktionszone C eine Temperatur im Bereich 230 bis 320°C und die Temperatur der Reaktionszone D ebenfalls eine Temperatur im Bereich 230 bis 320°C ist, und deren Aktivmasse wenigstens ein die Elemente Mo und V enthaltendes Multimetalloxid ist, führt, dass sich die Reaktionszone C bis zu einem Umsatz des Acroleins von 45 bis 85 mol.-% erstreckt und der Acroleinumsatz bei einmaligem Durchgang durch die Festbettkatalysatorschüttung 2 ≥90 mol.-% und die Selektivität der über alle Reaktionszonen bilanzierten Acrylsäurebildung, bezogen auf umgesetztes Propen, ≥80 mol.-% beträgt, wobei die zeitliche Abfolge, in der das Reaktionsgasgemisch die Reaktionszonen durchströmt der alphabetischen Abfolge der Reaktionszonen entspricht.

Acrylsäure ist ein bedeutendes Monomer, das als solches oder in Form seiner Acrylester zur Erzeugung von z.B. als Klebstoffen geeigneten Polymerisaten Verwendung findet.

Großtechnisch wird Acrylsäure überwiegend durch heterogen katalysierte partielle Gasphasenoxidation von Propen hergestellt (vgl. z.B. WO 01/36364).

Prinzipiell läuft die heterogen katalysierte partielle Gasphasenoxidation von Propen zu Acrylsäure in zwei zeitlich aufeinanderfolgenden Schritten ab.

Im ersten Schritt erfolgt die Oxidation des Propens zum Acrolein und im zweiten Schritt wird das im ersten Schritt gebildete Acrolein zu Acrylsäure oxidiert.

Üblicherweise wird die heterogen katalysierte partielle Gasphasenoxidation von Propen zu Acrylsäure daher bei erhöhten Temperaturen in zwei räumlich aufeinander folgenden Reaktionsstufen durchgeführt, wobei in jeder der beiden Reaktionsstufen katalytisch wirksame Multimetalloxidmassen verwendet werden, die auf den in der jeweiligen Reaktionsstufe zu katalysierenden Oxidationsschritt zugeschnitten sind. Die in der ersten Reaktionsstufe für den Schritt vom Propen zum Acrolein zu verwendenden Multimetalloxidaktivmassen sind dadurch charakterisiert, dass sie die Elemente Mo, Fe und Bi enthalten, während die in der zweiten Reaktionsstufe für den Schritt vom Acrolein zur Acrylsäure zu verwendenden Multimetalloxidaktivmassen dadurch charakterisiert sind, dass sie die Elemente Mo und V enthalten. Eine Zwischenabtrennung des Acroleins aus dem Produktgasgemisch der ersten Reaktionsstufe wird dabei normalerweise nicht vorgenommen. Vielmehr wird das Acrolein enthaltende Produktgasgemisch der ersten Reaktionsstufe, gegebenenfalls nach Verringerung seiner Temperatur durch Kühlung sowie gegebenenfalls nach Zusatz von weiterem molekularem Sauerstoff und/oder Inertgas, in der Regel als solches für die Weiteroxidation des Acroleins in der zweiten Reaktionsstufe verwendet.

Neben molekularem Sauerstoff und den Reaktanden enthält das Reaktionsgasgemisch u.a. deshalb Inertgas, um das Reaktionsgasgemisch außerhalb des Explosionsbereiches zu halten.

Eine Zielsetzung einer solchen zweistufigen heterogen katalysierten partiellen Gasphasenoxidation von Propen zu Acrylsäure besteht darin, bei einmaligem Durchgang des Reaktionsgasgemisches durch die beiden Reaktionsstufen unter ansonsten vorgegebenen Randbedingungen eine möglichst hohe Ausbeute A^{AA} an Acrylsäure zu erzielen (das ist die Molzahl von zu Acrylsäure umgesetztem Propen, bezogen auf die Molzahl an eingesetztem Propen).

Eine weitere Zielsetzung einer solchen heterogen katalysierten partiellen Gasphasenoxidation von Propen zu Acrylsäure besteht darin, eine möglichst hohe Raum-Zeit-Ausbeute RZA^{AA} an Acrylsäure zu erzielen (das ist bei einer kontinuierlichen Verfahrensweise die je Stunde und Gesamtvolumen der verwendeten Festbettkatalysatorschüttung in Litern erzeugte Gesamtmenge an Acrylsäure).

Bei gleichbleibender gegebener Ausbeute A^{AA} ist die Raum-Zeit-Ausbeute um so größer, je größer die Belastung der Festbettkatalysatorschüttung der ersten Reaktionsstufe (Festbettkatalysatorschüttung 1) mit Propen ist (darunter wird die Menge an Propen in Normlitern (= NI; das Volumen in Litern, das die entsprechende Propenmenge bei Normalbedingungen, d.h. bei 25°C und 1 bar, einnehmen würde) verstanden, die als Bestandteil des Reaktionsgasausgangsgemisches 1 pro Stunde durch einen Liter an Festbettkatalysatorschüttung 1 geführt wird).

Die Lehren der Schriften WO 01/36364, DE-A 19927624, DE-A 19948248, DE-A 19948523, DE-A 19948241 und DE-A 19910506 sind daher darauf gerichtet, bei im wesentlichen gleichbleibender A^{AA} die Propenbelastung der Festbettkatalysatorschüttung der ersten Reaktionsstufe signifikant zu erhöhen. Dies gelingt im wesentlichen dadurch, dass sowohl die Festbettkatalysatorschüttung in der ersten Reaktionsstufe als auch die Festbettkatalysatorschüttung in der zweiten Reaktionsstufe in jeweils zwei räumlich aufeinanderfolgenden Temperaturzonen (Reaktionszonen) angeordnet wird. Die Propenbelastung der Festbettkatalysatorschüttung 1 wird dabei zu ≥160 Nl/l Festbettkatalysatorschüttung 1 • h gewählt und die Temperatur der jeweils zweiten (in Strömungsrichtung des Reaktionsgasgemisches) Temperaturzone muss wenigstens 5°C oberhalb der Temperatur der ersten Temperaturzone liegen.

In ähnlicher Weise lehrt auch die EP-A 1106598 ein Verfahren der Hochlastfahrweise für die heterogen katalysierte partielle Gasphasenoxidation von Propen zu Acrylsäure, bei dem die Festbettkatalysatorschüttungen der beiden Reaktionsstufen jeweils in mehreren Temperaturzonen angeordnet sind. Dabei kann der Temperaturunterschied einer in Strömungsrichtung des Reaktionsgasgemisches nachfolgenden Temperaturzone gemäß der Lehre der EP-A 1106598 sowohl mehr als auch weniger als 5°C oberhalb der Temperatur der vorgehenden Temperaturzone liegen, wobei es die EP-A 1106598 völlig offen lässt, unter welchen Bedingungen ein größerer und unter welchen Bedingungen ein kleiner Temperaturunterschied angewendet werden sollte.

Auch lässt die EP-A 1106598 völlig offen, was unter der Temperatur einer Reaktions-bzw. Temperaturzone zu verstehen ist.

Im Unterschied dazu wird in den übrigen Schriften des zitierten Standes der Technik unter der Temperatur einer Reaktionszone die Temperatur der in der Reaktionszone befindlichen Festbettkatalysatorschüttung bei Ausübung des Verfahrens in Abwesenheit einer chemischen Reaktion verstanden. Ist diese Temperatur innerhalb der Reaktionszone nicht konstant, so meint der Begriff Temperatur einer Reaktionszone den (Zahlen)mittelwert der Temperatur der Festbettkatalysatorschüttung längs der Reaktionszone. Wesentlich ist dabei, dass die Temperierung der einzelnen Reaktionszonen im wesentlichen unabhängig voneinander erfolgt, weshalb einer Reaktionszone stets eine Temperaturzone entspricht. Vorstehende Definition der Temperatur einer Reaktionszone soll auch in dieser Schrift gelten.

Da die heterogen katalysierte partielle Gasphasenoxidation von Propen zu Acrylsäure eine ausgeprägt exotherme Reaktion ist, ist die Temperatur des Reaktionsgasgemisches beim reaktiven Durchgang durch die Festbettkatalysatorschüttung in der Regel von der Temperatur einer Reaktionszone verschieden. Sie liegt normalerweise oberhalb der Temperatur der Reaktionszone und durchläuft innerhalb einer Reaktionszone in der Regel ein Maximum (Heißpunktmaximum) oder fällt von einem Maximalwert ausgehend ab.

Nachteilig an den Lehren des Standes der Technik ist jedoch, dass sie ausschließlich darauf ausgerichtet sind, eine Mehrzonenanordnung unter hoher Propenlast zu betreiben. Dies ist insofern nachteilig, als mit einer solchen Verfahrensweise unabdingbar eine hohe RZA^{AA} einhergeht. Diese setzt aber einen entsprechenden Marktbedarf an Acrylsäure voraus. Ist letzterer nicht gegeben (z.B. temporär), muss die Mehrzonenanordnung in notwendiger Weise bei geringeren Propenlasten betrieben werden, wobei dann außerdem eine möglichst hohe Selektivität der Acrylsäurebildung, bezogen auf umgesetztes Propen, als anzustrebende Zielgröße im Vordergrund steht (S^{AA}). Das ist die bei einmaligem Durchgang durch die Mehrzonenanordnung gebildete molare Menge an Acrylsäure bezogen auf die Molzahl an dabei umgesetztem Propen.

Die Aufgabe der vorliegenden Erfindung bestand daher darin, ein Verfahren der heterogen katalysierten partiellen Gasphasenoxidation von Propen zu Acrylsäure in einer Mehrzonenanordnung zur Verfügung zu stellen, bei dem bei Propenlasten von < 160 NI/l • h die Acrylsäurebildung mit möglichst hoher Selektivität erfolgt.

Demgemäss wurde ein Verfahren der heterogen katalysierten partiellen Gasphasenoxidation von Propen zu Acrylsäure, bei dem man ein Propen, molekularen Sauerstoff und wenigstens ein Inertgas enthaltendes Reaktionsgasausgangsgemisch 1, das den molekularen Sauerstoff und das Propen in einem molaren Verhältnis O₂ : C₃H₆ ≥ 1 enthält, in einer ersten Reaktionsstufe so über eine Festbettkatalysatorschüttung 1, die in zwei räumlich aufeinanderfolgenden Reaktionszonen A, B angeordnet ist, wobei die Temperatur der Reaktionszone A eine Temperatur im Bereich von 290 bis 380°C und die Temperatur der Reaktionszone B ebenfalls eine Temperatur im Bereich von 290 bis 380°C ist, und deren Aktivmasse wenigstens ein die Elemente Mo, Fe und Bi enthaltendes Multimetalloxid ist, führt, dass ich die Reaktionszone A bis zu einem Umsatz des Propens von 40 bis 80 mol.-% erstreckt und der Propenumsatz bei einmaligem Durchgang durch die Festbettkatalysatorschüttung 1 ≥90 mol.-% und die damit einhergehende Selektivität der Acroleinbildung sowie der Acrylsäurenebenproduktbildung zusammengenommen ≥90 mol.-% betragen, die Temperatur des die erste Reaktionsstufe verlassenden Produktgasgemisches durch Kühlung gegebenenfalls verringert und dem Produktgasgemisch gegebenenfalls molekularen Sauerstoff und/oder Inertgas zugibt, und danach das Produktgasgemisch als Acrolein, molekularen Sauerstoff und wenigstens ein Inertgas enthaltendes Reaktionsgasausgangsgemisch 2, das den molekularen Sauerstoff und das Acrolein in einem molaren Verhältnis O₂ : C₃H₄O ≥0,5 enthält, in einer zweiten Reaktionsstufe so über eine Festbettkatalysatorschüttung 2, die in zwei räumlich aufeinanderfolgenden Reaktionszonen C, D angeordnet ist, wobei die Temperatur der Reaktionszone C eine Temperatur im Bereich 230 bis 320°C und die Temperatur der Reaktionszone D ebenfalls eine Temperatur im Bereich 230 bis 320°C ist, und deren Aktivmasse wenigstens ein die Elemente Mo und V enthaltendes Multimetalloxid ist, führt, dass sich die Reaktionszone C bis zu einem Umsatz des Acroleins von 45 bis 85 mol.% erstreckt und der Acroleinumsatz bei einmaligem Durchgang durch die Festbettkatalysatorschüttung 2 ≥90 mol.-% und die Selektivität der über alle Reaktionszonen bilanzierten Acrylsäurebildung, bezogen auf umgesetztes Propen, ≥80 mol.% beträgt, wobei die zeitliche Abfolge, in der das Reaktionsgasgemisch die Reaktionszonen durchströmt der alphabetischen Abfolge der Reaktionszonen entspricht, das dadurch gekennzeichnet ist, dass
a) die Belastung der Festbettkatalysatorschüttung 1 mit dem im Reaktionsgasausgangsgemisch 1 enthaltenen Propen < 160 NI Propen / I Festbettkatalysatorschüttung 1 • h und ≥90 NI Propen / I Festbettkatalysatorschüttung 1 • h beträgt;
b) die volumenspezifische Aktivität der Festbettkatalysatorschüttung 1 in Strömungsrichtung des Reaktionsgasgemisches über die Festbettkatalysatorschüttung 1 entweder konstant ist oder wenigstens einmal zunimmt;
c) die Differenz T^{maxA} - T^{maxB}, gebildet aus der höchsten Temperatur T^{maxA}, die das Reaktionsgasgemisch innerhalb der Reaktionszone A aufweist, und der höchsten Temperatur T^{maxB}, die das Reaktionsgasgemisch innerhalb der Reaktionszone B aufweist, ≥0°C beträgt,
d) die Belastung der Festbettkatalysatorschüttung 2 mit dem im Reaktionsgasausgangsgemisch 2 enthaltenen Acrolein ≤145 Nl Acrolein / I Festbettkatalysatorschüttung 2 • h und ≥70 Nl Acrolein /l Festbettkatalysatorschüttung 2 • h beträgt,
e) die volumenspezifische Aktivität der Festbettkatalysatorschüttung 2 in Strömungsrichtung des Reaktionsgasgemisches über die Festbettkatalysatorschüttung 2 wenigstens einmal zunimmt; und
f) die Differenz T^{maxC} - T^{maxD}, gebildet aus der höchsten Temperatur T^{maxC}, die das Reaktionsgasgemisch innerhalb der Reaktionszone C aufweist, und der höchsten Temperatur T^{maxD}, die das Reaktionsgasgemisch innerhalb der Reaktionszone D aufweist, ≥0°C beträgt.
In der Regel wird beim erfindungsgemäßen Verfahren die Differenz T^{maxA} - T^{maxB} nicht mehr als 80°C betragen.

Erfindungsgemäß bevorzugt, beträgt T^{maxA} - T^{maxB} ≥3°C und ≤70°C. Ganz besonders bevorzugt beträgt T^{maxA} - T^{maxB} beim erfindungsgemäßen Verfahren ≥20°C und ≤60°C.

Ferner wird die Differenz T^{maxC} - T^{maxD} beim erfindungsgemäßen Verfahren in der Regel nicht mehr als 75°C betragen.

Erfindungsgemäß bevorzugt beträgt T^{maxC} - T^{maxD} ≥3°C und ≤60°C. Ganz besonders bevorzugt beträgt T^{maxC} - T^{maxD} beim erfindungsgemäßen Verfahren ≥5°C und ≤40°C.

Das erfindungsgemäße Verfahren erweist sich z.B. als vorteilhaft, wenn die Belastung der Festbettkatalysatorschüttung 1 mit dem im Reaktionsgasausgangsgemisch 1 enthaltenen Propen ≥90 Nl Propen/l • h und ≤155 Nl Propen/l • h, oder ≥100 Nl Propen/l • h und ≤150 Nl Propen/l •h, oder ≥110 Nl Propen/l • h und ≤145 Nl Propen/l • h, oder ≥120 Nl Propen/l • h und ≤140 Nl Propen/l • h, oder ≥125 Nl Propen/l • h und ≤ 135 Nl Propen/l• h, und die Belastung der Festbettkatalysatorschüttung 2 mit dem im Reaktionsgasausgangsgemisch 2 enthaltenen Acrolein ≥70 Nl Acrolein/l • h und ≤ 140 Nl Acrolein/l • h bzw. ≤135 Nl Acrolein/l• h, oder ≥80 Nl Acrolein/l • h und ≤130 Nl Acrolein/l • h, oder ≥90 Nl Acrolein/l • h und ≤125 Nl Acrolein/l • h, oder ≥100 Nl Acrolein/l • h und ≤120 Nl Acrolein/l • h, oder ≥105 Nl Acrolein/l • h und ≤115 Nl Acrolein/l • h beträgt.

Selbstverständlich kann das erfindungsgemäße Verfahren auch dann angewendet werden, wenn die Belastung der Festbettkatalysatorschüttung 1 mit dem im Reaktionsgasausgangsgemisch 1 enthaltenen Propen < 90 NI Propen/l • h und die Belastung der Festbettkatalysatorschüttung 2 mit dem im Reaktionsgasausgangsgemisch 2 enthaltenen Acrolein < 70 NI Acrolein/l • h beträgt. Der Betrieb einer Mehrzonenanordnung bei solchermaßen geringen Reaktandenlasten wäre jedoch kaum noch wirtschaftlich.

Die erfindungsgemäß geforderten Differenzen T^{maxA} - T^{maxB} stellen sich bei der Ausübung des erfindungsgemäßen Verfahrens normalerweise dann ein, wenn einerseits sowohl die Temperatur der Reaktionszone A als auch die Temperatur der Reaktionszone B im Bereich von 290 bis 380°C liegt und andererseits die Differenz zwischen der Temperatur der Reaktionszone B (T_{B}) und der Temperatur der Reaktionszone A (T_{A}), d.h., T_{B}- T_{A}, ≤0°C und ≥-20°C oder ≤10°C, bzw. ≤0°C und ≥-5°C, oder häufig ≤0°C und ≥-3°C beträgt.

D.h., im Gegensatz zur Lehre des Standes der Technik für hohe Lasten, wird beim erfindungsgemäßen Verfahren die Temperatur der Nachfolgezone normalerweise geringer sein als die Temperatur der vorausgehenden Reaktionszone.

Dies gilt so auch für die beiden Reaktionszonen der zweiten Reaktionsstufe.

D.h., die erfindungsgemäß geforderten Differenzen T^{maxC} - T^{maxD} stellen sich bei der Ausübung des erfindungsgemäßen Verfahrens normalerweise dann ein, wenn einerseits sowohl die Temperatur der Reaktionszone C als auch die Temperatur der Reaktionszone D im Bereich von 230 bis 320°C liegt und andererseits die Differenz zwischen der Temperatur der Reaktionszone D (T_{D}) und der Temperatur der Reaktionszone C (T_{C}), d.h. T_{D} - T_{C}, ≤0°C und ≥-20°C oder ≥-10°C, bzw. ≤0°C und ≥-5°C, oder häufig ≤0°C und ≥-3°C beträgt.

Die vorgenannte Aussage betreffend die Temperaturdifferenz T_{B}- T_{A} gilt auch dann, wenn die Temperatur der Reaktionszone A im bevorzugten Temperaturbereich von 305 bis 365°C bzw. im besonders bevorzugten Bereich von 310 bis 340°C liegt.

In entsprechender Weise gilt die vorgenannte Aussage betreffend die Temperaturdifferenz T_{D} - T_{c} auch dann, wenn die Temperatur der Reaktionszone C im bevorzugten Temperaturbereich von 250 bis 300°C bzw. im besonders bevorzugten Bereich von 260°C bis 280°C liegt.

Der Arbeitsdruck kann in beiden Reaktionsstufen des erfindungsgemäßen Verfahrens sowohl unterhalb von Normaldruck (z.B. bis zu 0,5 bar) als auch oberhalb von Normaldruck liegen. Typischerweise wird der Arbeitsdruck in den beiden Reaktionsstufen des erfindungsgemäßen Verfahrens bei Werten von 1 bis 5 bar, häufig 1 bis 3 bar liegen.

Normalerweise wird der Reaktionsdruck in keiner der beiden Reaktionsstufen 100 bar überschreiten.

In der Regel wird der auf den einfachen Durchgang der Festbettkatalysatorschüttung 1 bezogene Propenumsatz beim erfindungsgemäßen Verfahren ≥92 mol-% oder ≥94 mol-% betragen.

Die Selektivität der Wertproduktbildung (Summe aus Acroleinbildung und Acrylsäurenebenproduktbildung) wird dabei bei in an sich bekannter Weise geeigneter Katalysatorwahl regelmäßig ≥92 mol-%, oder ≥94 mol-%, häufig ≥95 mol-%, oder ≥ 96 mol-% bzw. ≥97 mol-% betragen.

Erfindungsgemäß bevorzugt erstreckt sich die Reaktionszone A bis zu einem Propenumsatz von 50 bis 70 mol-% und besonders bevorzugt bis zu einem Propenumsatz von 60 bis 70 mol-%.

In der Regel wird beim erfindungsgemäßen Verfahren die Acroleinbelastung der Festbettkatalysatorschüttung 2 etwa 10 NI/l • h, häufig etwa 20 bzw. 25 NI/l • h unterhalb der Propenbelastung der Festbettkatalysatorschüttung 1 liegen. Dies ist primär darauf zurückzuführen, dass in der ersten Reaktionsstufe sowohl der Umsatz des Propens als auch die Selektivität der Acroleinbildung in der Regel keine 100 % erreichen.

Erfindungsgemäß bevorzugt erstreckt sich die Reaktionszone C bis zu einem Umsatz des aus der ersten Reaktionsstufe kommenden Acroleins von 50 bis 85 mol-% bzw. 60 bis 85 mol%.

In der Regel wird der auf den einfachen Durchgang der Festbettkatalysatorschüttung 2 bezogene Acroleinumsatz beim erfindungsgemäßen Verfahren ≥92 mol-% oder ≥94 mol-% oder ≥96 mol-% oder ≥98 mol-% und häufig sogar ≥99 mol-% oder mehr betragen.

Bei in an sich bekannter Weise geeigneter Wahl der Festbettkatalysatorschüttung 2 kann beim erfindungsgemäßen Verfahren die über beide Reaktionsstufen bilanzierte Selektivität der Acrylsäurebildung, bezogen auf umgesetztes Propen, bei Werten ≥83 mol-%, häufig bei ≥85 mol-%, oder ≥88 mol-%, oft bei ≥90 mol%, oder ≥93 mol-% liegen.

Das molare Verhältnis von O₂: C₃H₆ im Reaktionsgasausgangsgemisch 1 muss erfindungsgemäß ≥1 betragen. Üblicherweise wird dieses Verhältnis bei Werten ≤3 liegen.

Häufig beträgt das molare Verhältnis von O₂: C₃H₆ im Reaktionsgasausgangsgemisch 1 erfindungsgemäß ≥1,5 und ≤2,0.

Das molare Verhältnis von O₂: C₃H₄O im Reaktionsgasausgangsgemisch 2 beträgt erfindungsgemäß bevorzugt ebenfalls ≥1. Üblicherweise wird dieses Verhältnis ebenfalls bei Werten ≤3 liegen. Häufig wird das molare Verhältnis von O₂ : Acrolein im Reaktionsgasausgangsgemisch 2 erfindungsgemäß 1 bis 2 bzw. 1 bis 1,5 betragen.

Als Katalysatoren für die Festbettkatalysatorschüttung 1 des erfindungsgemäßen Verfahrens kommen alle diejenigen in Betracht, deren Aktivmasse wenigstens ein Mo, Bi und Fe enthaltendes Multimetalloxid ist.

Dies sind insbesondere die Multimetalloxidaktivmassen der allgemeinen Formel I der DE-A 19955176, die Multimetalloxidaktivmassen der allgemeinen Formel I der DE-A 19948523, die Multimetalloxidaktivmassen der allgemeinen Formeln I, II und III der DE-A 10101695, die Multimetalloxidaktivmassen der allgemeinen Formeln I, II und III der DE-A 19948248 und die Multimetalloxidaktivmassen der allgemeinen Formeln I, II und III der DE-A 19955168 sowie die in der EP-A 700714 genannten Multimetalloxidaktivmassen.

Ferner eignen sich für die Festbettkatalysatorschüttung 1 die Mo, Bi und Fe enthaltenden Multimetalloxidkatalysatoren die in den Schriften DE-A 10046957, DE-A 10063162, DE-C 3338380, DE-A 19902562, EP-A 15565, DE-C 2380765, EP-A 807465, EP-A 279374, DE-A 3300044, EP-A 575897, US-A 4438217, DE-A 19855913, WO 98/24746, DE-A 19746210 (diejenigen der allgemeinen Formel II), JP-A 91/294239, EP-A 293224 und EP-A 700714 offenbart werden. Dies gilt insbesondere für die beispielhaften Ausführungsformen in diesen Schriften, unter denen jene der EP-A 15565, der EP-A 575897, der DE-A 19746210 und der DE-A 19855913 besonders bevorzugt werden. Besonders hervorzuheben sind in diesem Zusammenhang ein Katalysator gemäß Beispiel 1 c aus der EP-A 15565 sowie ein in entsprechender Weise herzustellender Katalysator, dessen Aktivmasse jedoch die Zusammensetzung Mo₁₂Ni_{6,5}Zn₂Fe₂Bi₁P_{0.0065}K_{0.06}Ox • 10 SiO₂ aufweist. Ferner sind hervorzuheben das Beispiel mit der laufenden Nr. 3 aus der DE-A 19855913 (Stöchiometrie: Mo₁₂Co₇Fe₃Bi_{0,6}K_{0,08}Si_{1,6}Ox) als Hohlzylindervollkatalysator der Geometrie 5 mm x 3 mm x 2 mm bzw. 5 mm x 2 mm x 2 mm (jeweils Außendurchmesser x Höhe x Innendurchmesser) sowie der Multimetalloxid II - Vollkatalysator gemäß Beispiel 1 der DE-A 19746210. Ferner wären die Multimetalloxid-Katalysatoren der US-A 4438217 zu nennen. Letzteres gilt insbesondere dann, wenn diese eine Hohlzylinder-Geometrie der Ausmaße 5,5 mm x 3 mm x 3,5 mm, oder 5 mm x 2 mm x 2 mm, oder 5 mm x 3 mm x 2 mm, oder 6 mm x 3 mm x 3 mm, oder 7 mm x 3 mm x 4 mm (jeweils Außendurchmesser x Höhe x Innendurchmesser) aufweisen. Ebenso eignen sich die Multimetalloxidkatalysatoren und Geometrien der DE-A 10101695 bzw. WO 02/062737.

Weiterhin sind das Beispiel 1 aus der DE-A 10046957 (Stöchiometrie: [Bi₂W₂O₉ x 2WO₃]_{0,5} • [Mo₁₂Co_{5,6}Fe_{2,94}Si_{1,59}K_{0,08}Oₓ]₁) als Hohlzylinder(Ring)vollkatalysator der Geometrie 5 mm x 3 mm x 2 mm bzw. 5 mm x 2 mm x 2 mm (jeweils Außendurchmesser x Länge x Innendurchmesser), sowie die Schalenkatalysatoren 1, 2 und 3 aus der DE-A 10063162 (Stöchiometrie: Mo₁₂Bi_{1,0}Fe₃Co₇Si_{1,6}K_{0.08}), jedoch als ringförmige Schalenkatalysatoren entsprechender Schalendicke und auf Trägerringe der Geometrie 5 mm x 3 mm x 1,5 mm bzw. 7 mm x 3 mm x 1,5 mm (jeweils Außendurchmesser x Länge x Innendurchmesser) aufgebracht geeignet.

Eine Vielzahl der für die Katalysatoren der Festbettkatalysatorschüttung 1 geeigneten Multimetalloxidaktivmassen lässt sich unter der allgemeinen Formel I

Mo₁₂BiₐFe_{b}X¹_{c}X²_{d}X³ₑX⁴_{f}Oₙ (I),

in der die Variablen nachfolgende Bedeutung aufweisen:
X¹ = Nickel und/oder Kobalt,
X² = Thallium, ein Alkalimetall und/oder ein Erdalkalimetall,
X³ = Zink, Phosphor, Arsen, Bor, Antimon, Zinn, Cer, Blei und/oder Wolfram,
X⁴ = Silicium, Aluminium, Titan und/oder Zirkonium,

a = 0,5 bis 5,
b = 0,01 bis 5, vorzugsweise 2 bis 4,
c = 0 bis 10, vorzugsweise 3 bis 10,
d = 0 bis 2, vorzugsweise 0,02 bis 2,
e = 0 bis 8, vorzugsweise 0 bis 5,
f = 0 bis 10 und
n = eine Zahl, die durch die Wertigkeit und Häufigkeit der von Sauerstoff verschiedenen Elemente in I bestimmt wird,
subsummieren.

Sie sind in an sich bekannter Weise erhältlich (siehe z.B. die DE-A 4023239) und werden üblicherweise in Substanz zu Kugeln, Ringen oder Zylindern geformt oder auch in Gestalt von Schalenkatalysatoren, d.h., mit der Aktivmasse beschichteten vorgeformten, inerten Trägerkörpern, eingesetzt. Selbstverständlich können sie aber auch in Pulverform als Katalysatoren angewendet werden.

Prinzipiell können Aktivmassen der allgemeinen Formel 1 in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 650°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemisch aus Inertgas, NH₃, CO und/oder H₂) erfolgen. Die Calcinationsdauer kann einige Minuten bis einige Stunden betragen und nimmt üblicherweise mit der Temperatur ab. Als Quellen für die elementaren Konstituenten der Multimetalloxidaktivmassen I kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Neben den Oxiden kommen als solche Ausgangsverbindungen vor allem Halogenide, Nitrate, Formiate, Oxalate, Citrate, Acetate, Carbonate, Aminkomplexe, AmmoniumSalze und/oder Hydroxide in Betracht (Verbindungen wie NH₄OH, (NH₄)₂CO₃, NH₄NO₃, NH₄CHO₂, CH₃COOH, NH₄CH₃CO₂ und/oder Ammoniumoxalat, die spätestens beim späteren Calcinieren zu gasförmig entweichenden Verbindungen zerfallen und/oder zersetzt werden können, können in das innige Trockengemisch zusätzlich eingearbeitet werden).

Das innige Vermischen der Ausgangsverbindungen zur Herstellung von Multimetalloxidaktivmassen I kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form. Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozeß vorzugsweise durch Sprühtrocknung der wässrigen Mischung mit Austrittstemperaturen von 100 bis 150°C erfolgt.

Üblicherweise werden die Multimetalloxidaktivmassen der allgemeinen Formel I in der Festbettkatalysatorschüttung 1 nicht in Pulverform sondern zu bestimmten Katalysatorgeometrien geformt eingesetzt, wobei die Formgebung vor oder nach der abschließenden Calcination erfolgen kann. Beispielsweise können aus der Pulverform der Aktivmasse oder ihrer uncalcinierten und/oder partiell calcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Eine besonders günstige Hohlzylindergeometrie beträgt 5 mm x 3 mm x 2 mm (Außendurchmesser x Länge x Innendurchmesser), insbesondere im Fall von Vollkatalysatoren.

Selbstverständlich kann die Formgebung der pulverförmigen Aktivmasse oder ihrer pulverförmigen, noch nicht und/oder partiell calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist. Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 *µ*m, bevorzugt im Bereich 50 bis 500 *µ*m und besonders bevorzugt im Bereich 150 bis 250 *µ*m liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Sie verhalten sich bezüglich der dem erfindungsgemäßen Verfahren in der ersten Reaktionsstufe unterliegenden Zielreaktion in der Regel im wesentlichen inert. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit (z.B. Steatit C220 der Fa. CeramTec), deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 2 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Für die Katalysatoren der ersten Reaktionsstufe geeignete Multimetalloxidaktivmassen sind ferner Massen der allgemeinen Formel II

[Y¹_{a'}Y²_{b'}O_{x'}]ₚ[Y³_{c'}Y⁴_{d'}Y⁵_{e'}Y⁶_{f'}Y⁷_{g'}Y²_{h'}O_{y'}]_{q} (II),

in der die Variablen folgende Bedeutung haben:
Y¹ = nur Wismut oder Wismut und wenigstens eines der Elemente Tellur, Antimon, Zinn und Kupfer,
Y² = Molybdän oder Molybdän und Wolfram,
Y³ = ein Alkalimetall, Thallium und/oder Samarium,
Y⁴= ein Erdalkalimetall, Nickel, Kobalt, Kupfer, Mangan, Zink, Zinn, Cadmium und/oder Quecksilber,
Y⁵ = Eisen oder Eisen und wenigstens eines der Elemente Chrom und Cer,
Y⁶ = Phosphor, Arsen, Bor und/oder Antimon,
Y⁷ = ein seltenes Erdmetall, Titan, Zirkonium, Niob, Tantal, Rhenium, Ruthenium, Rhodium, Silber, Gold, Aluminium, Gallium, Indium, Silicium, Germanium, Blei, Thorium und/oder Uran,

a' = 0,01 bis 8,
b' = 0,1 bis 30,
c' = 0 bis 4,
d' = 0 bis 20,
e' > 0 bis 20,
f' = 0 bis 6,
g' = 0 bis 15,
h' = 8 bis 16,
x',y'= Zahlen, die durch die Wertigkeit und Häufigkeit der von Sauerstoff verschiedenen Elemente in II bestimmt werden und
p,q = Zahlen, deren Verhältnis p/q 0,1 bis 10 beträgt,
enthaltend dreidimensional ausgedehnte, von ihrer lokalen Umgebung aufgrund ihrer von ihrer lokalen Umgebung verschiedenen Zusammensetzung abgegrenzte, Bereiche der chemischen Zusammensetzung Y¹_{a'}Y²_{b'}O_{x'}, deren Größtdurchmesser (längste durch den Schwerpunkt des Bereichs gehende Verbindungsstrecke zweier auf der Oberfläche (Grenzfläche) des Bereichs befindlicher Punkte) 1 nm bis 100 µm, häufig 10 nm bis 500 nm oder 1 µm bis 50 bzw. 25 µm, beträgt.

Besonders vorteilhafte erfindungsgemäße Multimetalloxidmassen II sind solche, in denen Y¹ nur Wismut ist.

Unter diesen werden wiederum jene bevorzugt, die der allgemeinen Formel III

[Bi_{a"}-Z²_{b"}-O_{x"}-]_{p"}- [Z²₁₂Z³_{c"}-Z⁴_{d"}-Fe_{e"}-Z⁵_{f"}-Z⁶_{g"}-Z⁷_{h"}-O_{y"}-]_{q"}- (III)

in der die Varianten folgende Bedeutung haben:
Z² = Molybdän oder Molybdän und Wolfram,
Z³ = Nickel und/oder Kobalt,
Z⁴ = Thallium, ein Alkalimetall und/oder ein Erdalkalimetall,
Z⁵ = Phosphor, Arsen, Bor, Antimon, Zinn, Cer und/oder Blei,
Z⁶ = Silicium, Aluminium, Titan und/oder Zirkonium,
Z⁷ = Kupfer, Silber und/oder Gold,

a" = 0,1 bis 1,
b" = 0,2 bis 2,
c" = 3 bis 10,
d" = 0,02 bis 2,
e" = 0,01 bis 5, vorzugsweise 0,1 bis 3,
f" = 0 bis 5,
g" = 0 bis 10,
h" = 0 bis 1,
x",y"= Zahlen, die durch die Wertigkeit und Häufigkeit der von Sauerstoff verschiedenen Element in III bestimmt werden,
p",q"=Zahlen, deren Verhältnis p"/q" 0,1 bis 5, vorzugsweise 0,5 bis 2 beträgt,
entsprechen, wobei diejenigen Massen III ganz besonders bevorzugt werden, in denen Z²_{b"} = (Wolfram)_{b"} und Z²₁₂ = (Molybdän)₁₂ ist.

Ferner ist es von Vorteil, wenn wenigstens 25 mol-% (bevorzugt wenigstens 50 mol-% und besonders bevorzugt wenigstens 100 mol-%) des gesamten Anteils [Y¹_{a'}Y²_{b'}O_{x'}]ₚ ([Biₐ-Z²_{b}-O_{x"}]_{p"}) der erfindungsgemäß geeigneten Multimetalloxidmassen II (Multimetalloxidmassen III) in den erfindungsgemäß geeigneten Multimetalloxidmassen II (Multimetalloxidmassen III) in Form dreidimensional ausgedehnter, von ihrer lokalen Umgebung aufgrund ihrer von ihrer lokalen Umgebung verschiedenen chemischen Zusammensetzung abgegrenzter, Bereiche der chemischen Zusammensetzung Y¹_{a'}Y²_{b'}O_{x'} [Bi_{a"}Z²_{b"}O_{x"}) vorliegen, deren Größtdurchmesser im Bereich 1 nm bis 100 *µ*m liegt.

Hinsichtlich der Formgebung gilt bezüglich Multimetalloxidmassen II-Katalysatoren das bei den Multimetalloxidmassen I-Katalysatoren Gesagte.

Die Herstellung von Multimetalloxidmassen II-Aktivmassen ist z.B. in der EP-A 575897 sowie in der DE-A 19855913 beschrieben.

Die vorstehend empfohlenen inerten Trägermaterialien kommen u.a. auch als inerte Materialien zur Verdünnung und/oder Abgrenzung des entsprechenden Katalysatorfestbetts, bzw. als deren schützende Vorschüttung in Betracht.

Für die Katalysatoren der Festbettkatalysatorschüttung 2 kommen prinzipiell alle Mo und V enthaltenden Multimetalloxidmassen als Aktivmassen in Betracht, z.B. jene der DE-A 10046928.

Eine Vielzahl derselben, z.B. diejenigen der DE-A 19815281, lässt sich unter der allgemeinen Formel IV

Mo₁₂VₐX¹_{b}X²_{c}X³_{d}X⁴ₑX⁵_{f}X⁶_{g}Oₙ (IV),

in der die Variablen folgende Bedeutung haben:
X¹ = W, Nb, Ta, Cr und/oder Ce,
X² = Cu, Ni, Co, Fe, Mn und/oder Zn,
X³ = Sb und/oder Bi,
X⁴ = eines oder mehrere Alkalimetalle,
X⁵ = eines oder mehrere Erdalkalimetalle,
X⁶ = Si, Al, Ti und/oder Zr,

a = 1 bis 6,
b = 0,2 bis 4,
c = 0,5 bis 18,
d = 0 bis 40,
e = 0 bis 2,
f = 0 bis 4,
g = 0 bis 40 und
n = eine Zahl, die durch die Wertigkeit und Häufigkeit der von Sauerstoff verschiedenen Elemente in IV bestimmt wird,
subsummieren.

Erfindungsgemäß bevorzugte Ausführungsformen innerhalb der aktiven Multimetalloxide IV sind jene, die von den nachfolgenden Bedeutungen der Variablen der allgemeinen Formel IV erfasst werden:
X¹ = W, Nb, und/oder Cr,
X² = Cu, Ni, Co, und/oder Fe,
X³ = Sb,
X⁴ = Na und/oder K,
X⁵ = Ca, Sr und/oder Ba,
X⁶ = Si, Al, und/oder Ti,

a = 1,5 bis 5,
b = 0,5 bis 2,
c = 0,5 bis 3,
d = 0 bis 2,
e = 0 bis 0,2,
f = 0 bis 1 und
n = eine Zahl, die durch die Wertigkeit und Häufigkeit der von Sauerstoff verschiedenen Elemente in IV bestimmt wird.

Erfindungsgemäß ganz besonders bevorzugte Multimetalloxide IV sind jedoch jene der allgemeinen Formel V

Mo₁₂V_{a'}Y¹_{b'}Y²_{c'}Y⁵_{f'}Y⁶_{g'}O_{n'} (V)

mit
Y¹ = W und/oder Nb,
Y² = Cu und/oder Ni,
Y⁵ = Ca und/oder Sr,
Y⁶ = Si und/oder Al,
a' = 2 bis 4,
b' = 1 bis 1,5,
c' = 1 bis 3,
f' = 0 bis 0,5
g'= 0 bis 8 und
n' = eine Zahl, die durch die Wertigkeit und Häufigkeit der von Sauerstoff verschiedenen Elementen in V bestimmt wird.

Die erfindungsgemäß geeigneten Multimetalloxidaktivmassen (IV) sind in an sich bekannter, z.B. in der DE-A 4335973 oder in der EP-A 714700 offenbarter, Weise erhältlich.

Prinzipiell können für die Katalysatoren der Festbettkatalysatorschüttung 2 geeignete Multimetalloxidaktivmassen, insbesondere solche der allgemeinen Formel IV, in einfacher Weise dadurch hergestellt werden, dass man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemische aus Inertgas und reduzierenden Gasen wie H₂, NH₃, CO, Methan und/oder Acrolein oder die genannten reduzierend wirkenden Gase für sich) durchgeführt werden. Die Calcinationsdauer'kann einige Minuten bis einige Stunden betragen und nimmt üblicherweise mit der Temperatur ab. Als Quellen für die elementaren Konstituenten der Multimetalloxidaktivmassen IV kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen IV kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wässrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wässrige Masse getrocknet, wobei der Trocknungsprozess vorzugsweise durch Sprühtrocknung der wässrigen Mischung mit Austrittstemperaturen von 100 bis 150°C erfolgt.

Die resultierenden Multimetalloxidmassen, insbesondere jene der allgemeinen Formel IV, werden in der Regel nicht in Pulverform sondern zu bestimmten Katalysatorgeometrien geformt in der Festbettkatalysatorschüttung 2 eingesetzt, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist.

Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 *µ*m, bevorzugt im Bereich 50 bis 500 *µ*m und besonders bevorzugt im Bereich 150 bis 250 *µ*m liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder mit Splittauflage, bevorzugt werden. Geeignet ist die Verwendung von im wesentlichen unporösen, oberflächenrauhen, kugelförmigen Trägern aus Steatit (z.B. Steatit C220 der Fa. CeramTec), deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von Ringen als Trägerkörper liegt die Wanddicke darüber hinaus üblicherweise bei 1 bis 4 mm. Bevorzugt zu verwendende ringförmige Trägerkörper besitzen eine Länge von 2 bis 6 mm, einen Außendurchmesser von 4 bis 8 mm und eine Wanddicke von 1 bis 2 mm. Geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepasst (vgl. EP-A 714 700).

Günstige für die Katalysatoren der Festbettkatalysatorschüttung 2 zu verwendende Multimetalloxidaktivmassen sind ferner Massen der allgemeinen Formel VI,

[D]ₚ[E]_{q} (VI),

in der die Variablen folgende Bedeutung haben:
D = Mo₁₂V_{a"}Z¹_{b"}Z²_{c"}Z³_{d"}Z⁴_{e"}Z⁵_{f"}Z⁶_{g"}O_{x"} ,
E = Z⁷₁₂Cu_{h"}H_{i"}O_{y"},
Z¹ = W, Nb, Ta, Cr und/oder Ce,
Z² = Cu, Ni, Co, Fe, Mn und/oder Zn,
Z³ = Sb und/oder Bi,
Z⁴ = Li, Na, K, Rb, Cs und/oder H
Z⁵ = Mg, Ca, Sr und/oder Ba,
Z⁶ = Si, Al, Ti und/oder Zr,
Z⁷ = Mo, W, V, Nb und/oder Ta, vorzugsweise Mo und/oder W

a" = 1 bis 8,
b" = 0,2 bis 5,
c" = 0 bis 23,
d" = 0 bis 50,
e" = 0 bis 2,
f" = 0 bis 5,
g" = 0 bis 50,
h" = 4 bis 30,
i" = 0 bis 20 und
x",y" = Zahlen, die durch die Wertigkeit und Häufigkeit der von Sauerstoff verschiedenen Element in VI bestimmt werden und
p,q = von Null verschiedene Zahlen, deren Verhältnis p/q 160:1 bis 1:1 beträgt,
und die dadurch erhältlich sind, dass man eine Multimetalioxidmasse E

Z⁷₁₂Cu_{h"}-H_{i"}-O_{y"} (E),

in feinteiliger Form getrennt vorbildet (Ausgangsmasse 1) und anschließend die vorgebildete feste Ausgangsmasse 1 in eine wässrige Lösung, eine wässrige Suspension oder in ein feinteiliges Trockengemisch von Quellen der Elemente Mo, V, Z¹, Z², Z³, Z⁴, Z⁵, Z⁶, die die vorgenannten Elemente in der Stöchiometrie D

Mo¹²V_{a"}Z¹_{b"}Z²_{c"}Z³_{d"}Z⁴_{e"}Z⁵_{f"}Z⁶_{g"} (D),

enthält (Ausgangsmasse 2), im gewünschten Mengenverhältnis p:q einarbeitet, die dabei gegebenenfalls resultierende wässrige Mischung trocknet, und die so gegebene trockene Vorläufermasse vor oder nach ihrer Trocknung zur gewünschten Katalysatorgeometrie bei Temperaturen von 250 bis 600°C calciniert.

Bevorzugt sind die Multimetalloxidmassen VI, bei denen die Einarbeitung der vorgebildeten festen Ausgangsmasse 1 in eine wässrige Ausgangsmasse 2 bei einer Temperatur < 70°C erfolgt. Eine detaillierte Beschreibung der Herstellung von Multimetalloxidmassen VI-Katalysatoren enthalten z.B. die EP-A 668104, die DE-A 19736105, die DE-A 10046928, die DE-A 19740493 und die DE-A 19528646.

Hinsichtlich der Formgebung gilt bezüglich Multimetalloxidmassen VI-Katalysatoren das bei den Multimetalloxidmassen IV-Katalysatoren Gesagte.

Darüber hinaus eignen sich als für die Katalysatoren der Festbettkatalysatorschüttung 2 geeignete Multimetalloxidmassen jene der DE-A 19815281, insbesondere alle beispielhaften Ausführungsformen aus dieser Schrift. Hinsichtlich der Formgebung gilt das vorstehend Gesagte.

Für die Festbettkatalysatorschüttung 2 des erfindungsgemäßen Verfahrens besonders geeignete Katalysatoren sind die Schalenkatalysatoren S1 (Stöchiometrie: Mo₁₂V₃W_{1,2}Cu_{2,4}Oₙ) und S7 (Stöchiometrie: Mo₁₂V₃W_{1,2}Cu_{1,6}Ni_{0,8}Oₙ) aus der DE-A 4442346 mit einem Aktivmassenanteil von 27 Gew.-% und einer Schalendicke von 230 *µ*m, der Schalenkatalysator aus Herstellungsbeispiel 5 der DE-A 10046928 (Stöchiometrie: Mo₁₂V₃W_{1,2}Cu_{2,4}Oₙ) mit einem Aktivmassenanteil von 20 Gew.-%, die Schalenkatalysatoren gemäß den Beispielen 1 bis 5 aus der DE-A 19815281, jedoch ebenso wie die vorstehend genannten Schalenkatalysatoren für die zweite Reaktionsstufe auf Trägerringe der Geometrie 7 mm x 3 mm x 4 mm (Außendurchmesser x Länge x Innendurchmesser) mit einem Aktivmassenanteil von 20 Gew.-% (bezogen auf die Gesamtmasse des Schalenkatalysators) aufgebracht, sowie ein Schalenkatalysator mit zweiphasiger Aktivmasse der Stöchiometrie (Mo_{10,4}V₃W_{1,2}Oₓ) (CuMo_{0,5}W_{0,5}O₄)_{1,6}, und hergestellt gemäß der DE-A 19736105 und einem Aktivmassenanteil von 20 Gew.-% auf den vorgenannten 7 mm x 3 mm x 4 mm Träger aufgebracht.

Die vorstehend für die zweite Reaktionsstufe empfohlenen Katalysatoren sind für die zwei Reaktionsstufen aber auch dann geeignet, wenn man alles beibehält und nur die Trägergeometrie auf 5 mm x 3 mm x 1,5 mm abändert (Außendurchmesser x Länge x Innendurchmesser). Ferner können die genannten Multimetalloxide aber auch in Form der entsprechenden Vollkatalysatorringe in der zweiten Reaktionsstufe eingesetzt werden.

Zur Bereitung der Festbettkatalysatorschüttung 1 können beim erfindungsgemäßen Verfahren nur die entsprechende Multimetalloxidaktivmasse aufweisende Katalysatorformkörper oder auch weitgehend homogene Gemische aus Multimetalloxidaktivmasse aufweisenden Katalysatorformkörpern und keine Multimetalloxidaktivmasse aufweisenden, sich bezüglich der heterogen katalysierten partiellen Gasphasenoxidation im wesentlichen inert verhaltenden, Formkörpern (Verdünnungsformkörper) verwendet werden. Als Materialien für solche inerten Formkörper kommen prinzipiell alle diejenigen in Betracht, die sich auch als Trägermaterial für erfindungsgemäß geeignete Schalenkatalysatoren eignen. Als solche Materialien kommen z.B. poröse oder unporöse Aluminiumoxide, Siliciumdioxid, Thoriumdioxid, Zirkondioxid, Siliciumcarbid, Silikate wie Magnesium- oder Aluminiumsilikat oder der bereits erwähnte Steatit (z.B. Steatit C-220 der Fa. CeramTec) in Betracht.

Die Geometrie solcher inerter Verdünnungsformkörper kann im Prinzip beliebig sein. D.h., es können beispielsweise Kugeln, Polygone, Vollzylinder oder auch Ringe sein. Erfindungsgemäß bevorzugt wird man als inerte Verdünnungskörper solche wählen, deren Geometrie derjenigen der mit ihnen zu verdünnenden Katalysatorformkörper entspricht (vorgenannte Aussagen gelten so auch für zur Bereitung der Festbettkatalysatorschüttung 2 verwendbare weitgehend homogene Gemische aus entsprechende Multimetalloxidaktivmasse aufweisenden Katalysatorformkörpern und Verdünnungsformkörpern).

Erfindungsgemäß günstig ist es, wenn sich die chemische Zusammensetzung der verwendeten Aktivmasse über die Festbettkatalysatorschüttung 1 nicht verändert. D.h., die für einen einzelnen Katalysatorformkörper verwendete Aktivmasse kann zwar ein Gemisch aus verschiedenen die Elemente Mo, Fe und Bi enthaltenden Multimetalloxiden sein, für alle Katalysatorformkörper der Festbettkatalysatorschüttung 1 ist dann jedoch das gleiche Gemisch zu verwenden.

Die volumenspezifische (d.h., die auf die Einheit des Volumens normierte) Aktivität kann in diesem Fall z.B. in einfacher Weise dadurch verringert werden, dass man eine Grundmenge von einheitlich hergestellten Katalysatorformkörpern mit Verdünnungsformkörpern homogen verdünnt. Je höher der Anteil der Verdünnungsformkörper g e-wählt wird, desto geringer ist die in einem bestimmten Volumen der Schüttung enthaltene Aktivmasse bzw. Katalysatoraktivität.

Eine in Strömungsrichtung des Reaktionsgasgemisches über die Festbettkatalysatorschüttung 1 wenigstens einmal zunehmende volumenspezifische Aktivität lässt sich für das erfindungsgemäße Verfahren somit in einfacher Weise z.B. dadurch einstellen, dass man die Schüttung mit einem hohen Anteil an inerten Verdünnungsformkörpern bezogen auf eine Sorte von Katalysatorformkörpern beginnt, und dann diesen Anteil an Verdünnungsformkörpern in Strömungsrichtung entweder kontinuierlich oder wenigstens einmal oder mehrfach abrupt (z.B. stufenförmig) verringert. Lässt man den Anteil an Verdünnungsformkörpern konstant oder werden überhaupt keine Verdünnungsformkörper in der Festbettkatalysatorschüttung 1 mitverwendet, resultiert dann eine konstante volumenspezifische Aktivität in Strömungsrichtung des Reaktionsgasgemisches über die Festbettkatalysatorschüttung 1. Eine Zunahme der volumenspezifischen Aktivität ist aber auch z.B. dadurch möglich, dass man bei gleichbleibender Geometrie und Aktivmassenart eines Schalenkatalysatorformkörpers die Dicke der auf dem Träger aufgebrachten Aktivmassenschicht erhöht oder in einem Gemisch aus Schalenkatalysatoren mit gleicher Geometrie aber mit unterschiedlichem Gewichtsanteil der Aktivmasse den Anteil an Katalysatorformkörpern mit höherem Aktivmassengewichtsanteil steigert. Alternativ kann man auch die Aktivmassen selbst verdünnen, indem man bei der Aktivmassenherstellung z.B. in das zu calcinierende Trockengemisch aus Ausgangsverbindungen inerte verdünnend wirkende Materialien wie hochgebranntes Siliciumdioxid einarbeitet. Unterschiedliche Zusatzmengen an verdünnend wirkendem Material führen automatisch zu unterschiedlichen Aktivitäten. Je mehr verdünnend wirkendes Material zugesetzt wird, desto geringer wird die resultierende Aktivität sein. Analoge Wirkung lässt sich auch z.B. dadurch erzielen, dass man in Mischungen aus Vollkatalysatoren und aus Schalenkatalysatoren (bei identischer Aktivmasse) in entsprechender Weise das Mischungsverhältnis verändert. Selbstredend lassen sich die beschriebenen Varianten auch kombiniert anwenden.

Natürlich können für die Festbettkatalysatorschüttung 1 aber auch Mischungen aus Katalysatoren mit chemisch unterschiedlicher Aktivmassenzusammensetzung und als folge dieser verschiedenen Zusammensetzung unterschiedlicher Aktivität verwendet werden. Diese Mischungen können wiederum mit inerten Verdünnungskörpem verdünnt werden.

Im Normalfall wird beim erfindungsgemäßen Verfahren weder innerhalb der Festbettkatalysatorschüttung 1 noch innerhalb der Festbettkatalysatorschüttung 2 in Strömungsrichtung des Reaktionsgasgemisches die volumenspezifische Aktivität einmal abnehmen.

Vorab und/oder im Anschluss an die Festbettkatalysatorschüttung 1 können sich ausschließlich aus Inertmaterial (z.B. nur Verdünnungsformkörpem) bestehende Schüttungen befinden (sie werden in dieser Schrift begrifflich nicht der Festbettkatalysatorschüttung 1 zugerechnet, da sie keine Formkörper enthalten, die Multimetalloxidaktivmasse aufweisen). Dabei können die für die Inertschüttung verwendeten Verdünnungsformkörper die gleiche Geometrie wie die in der Festbettkatalysatorschüttung 1 verwendeten Katalysatorformkörper aufweisen. Die Geometrie der für die Inertschüttung verwendeten Verdünnungsformkörper kann aber auch von der vorgenannten Geometrie der Katalysatorformkörper verschieden sein (z.B. kugelförmig anstatt ringförmig).

Häufig weisen die für solche Inertschüttungen verwendeten Formkörper die ringförmige Geometrie 7 mm x 7 mm x 4 mm (Außendurchmesser x Länge x Innendurchmesser) oder die kugelförmige Geometrie mit dem Durchmesser d = 4-5 mm auf.

Erfindungsgemäß bevorzugt ist beim erfindungsgemäßen Verfahren die Festbettkatalysatorschüttung 1 in Strömungsrichtung des Reaktionsgasgemisches wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h. z.B. auf einer Länge von 0,70 bis 1,50 m, bevorzugt 0,90 bis 1,20 m), jeweils der Gesamtlänge der Festbettkatalysatorschüttung 1, ein homogenes Gemisch aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 5 bis 40 Gew.-%, oder 10 bis 40 Gew.%, oder 20 bis 40 Gew.-%, oder 25 bis 35 Gew.% beträgt. Im Anschluss an diese erste Zone der Festbettkatalysatorschüttung 1 befindet sich dann erfindungsgemäß vorteilhaft bis zum Ende der Länge der Festbettkatalysatorschüttung 1 (d.h., z.B. ' auf einer Länge von 2,00 bis 3,00 m, bevorzugt 2,50 bis 3,00 m) entweder eine nur in geringerem Umfang (als in der ersten Zone) verdünnte Schüttung der Katalysatorformkörper, oder, ganz besonders bevorzugt, eine alleinige (unverdünnte) Schüttung derselben Katalysatorformkörper, die auch in der ersten Zone verwendet worden sind. Das Vorgenannte trifft insbesondere dann zu, wenn in der Festbettkatalysatorschüttung 1 als Katalysatorformkörper Vollkatalysatorringe oder Schalenkatalysatorringe (insbesondere jene, die in dieser Schrift als bevorzugt genannt werden) eingesetzt werden. Mit Vorteil weisen im Rahmen der vorgenannten Strukturierung sowohl die Katalysatorformkörper als auch die Verdünnungsformkörper beim erfindungsgemäßen Verfahren im wesentlichen die Ringgeometrie 5 mm x 3 mm x 2 mm (Außendurchmesser x Länge x Innendurchmesser) auf.

In entsprechender Weise wie die volumenspezifische Aktivität der Festbettkatalysatorschüttung 1 variiert werden kann, kann auch die volumenspezifische Aktivität der Festbettkatalysatorschüttung 2 variiert werden. Dabei kann sich vorab und/oder im Anschluss an die eigentliche Festbettkatalysatorschüttung 2 wiederum eine entsprechende Inertschüttung befinden. Eine Konstanz der volumenspezifischen Aktivität innerhalb der Festbettkatalysatorschüttung 2 (wie sie innerhalb der Festbettkatalysatorschüttung 1 erfindungsgemäß möglich ist) ist beim erfindungsgemäßen Verfahren jedoch ausgeschlossen.

Erfindungsgemäß bevorzugt ist beim erfindungsgemäßen Verfahren die Festbettkatalysatorschüttung 2 in Strömungsrichtung des Reaktionsgasgemisches wie folgt strukturiert.

Zunächst auf einer Länge von 10 bis 60 %, bevorzugt 10 bis 50 %, besonders bevorzugt 20 bis 40 % und ganz besonders bevorzugt 25 bis 35 % (d.h., z.B. auf einer Länge von 0,70 bis 1,50 m, bevorzugt 0,90 bis 1,20 m), jeweils der Gesamtlänge der Festbettkatalysatorschüttung 2, ein homogenes Gemisch oder zwei mit (abnehmender Verdünnung) aufeinanderfolgende homogene Gemische aus Katalysatorformkörpern und Verdünnungsformkörpern (wobei beide vorzugsweise im wesentlichen die gleiche Geometrie aufweisen), wobei der Gewichtsanteil der Verdünnungsformkörper (die Massendichten von Katalysatorformkörpern und von Verdünnungsformkörpern unterscheiden sich in der Regel nur geringfügig) normalerweise 10 bis 50 Gew.-%, vorzugsweise 20 bis 45 Gew.% und besonders bevorzugt 25 bis 35 Gew.-% beträgt. Im Anschluss an diese erste Zone der Festbettkatalysatorschüttung 2 befindet sich dann erfindungsgemäß vorteilhaft bis zum Ende der Länge der Festbettkatalysatorschüttung 2 (d.h., z.B. auf einer Länge von 2,00 bis 3,00 m, bevorzugt 2,50 bis 3,00 m) entweder eine nur in geringerem Umfang (als in der ersten Zone) verdünnte Schüttung der Katalysatorformkörper, oder, ganz besonders bevorzugt, eine alleinige Schüttung derselben Katalsatorformkörper, die auch in der ersten Zone verwendet worden sind.

Das Vorgenannte trifft insbesondere dann zu, wenn in der Festbettkatalysatorschüttung 2 als Katalysatorformkörper Schalenkatalysatorringe oder Schalenkatalysatorkugeln eingesetzt werden (insbesondere jene, die in dieser Schrift als bevorzugt angeführt werden). Mit Vorteil weisen im Rahmen der vorgenannten Strukturierung sowohl die Katalysatorformkörper bzw. deren Trägerringe als auch die Verdünnungsformkörper beim erfindungsgemäßen Verfahren im wesentlichen die Ringgeometrie 7 mm x 3 mm x 4 mm (Außendurchmesser x Länge x Innendurchmesser) auf.

Das oben Genannte trifft auch dann zu, wenn anstelle inerter Verdünnungskörper Schalenkatalysatorformkörper verwendet werden, deren Aktivmassenanteil um 2 bis 15 Gew.%-Punkte tiefer liegt, als der Aktivmassenanteil der Schalenkatalysatorformkörper am Ende der Festbettkatalysatorschüttung 2.

In anwendungstechnisch zweckmäßiger Weise erfolgt die Durchführung der ersten Reaktionsstufe des erfindungsgemäßen Verfahrens in einem Zweizonenrohrbündelreaktor, wie er z.B. in den DE-A's 19910508, 19948523, 19910506 und 19948241 beschrieben ist. Eine bevorzugte Variante eines erfindungsgemäß einsetzbaren Zweizonenrohrbündelreaktors offenbart die DE-C 2830765. Aber auch die in der DE-C 2513405, der US-A 3147084, der DE-A 2201528, der EP-A 383224 und der DE-A 2903218 offenbarten Zweizonenrohrbündelreaktoren sind für eine Durchführung der ersten Reaktionsstufe des erfindungsgemäßen Verfahrens geeignet.

D.h., in einfachster Weise befindet sich die erfindungsgemäß zu verwendende Festbettkatalysatorschüttung 1 (eventuell mit vor- und/oder nachangeordneten Inertschüttungen) in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Reaktionszone. D.h., in einfachster Weise umströmt z.B. ein Salzbad A denjenigen Abschnitt der Rohre (die Reaktionszone A), in welchem sich die oxidative Umsetzung des Propens (beim einfachen Durchgang) bis zum Erreichen eines Umsatzes im Bereich von 40 bis 80 mol-% vollzieht und ein Salzbad B umströmt den Abschnitt der Rohre (die Reaktionszone B), in welchem sich die oxidative Anschlussumsetzung des Propens (beim einfachen Durchgang) bis zum Erreichen eines Umsatzwertes von wenigstens 90 mol-% vollzieht (bei Bedarf können sich an die erfindungsgemäß anzuwendenden Reaktionszonen A,B weitere Reaktionszonen anschließen, die auf individuellen Temperaturen gehalten werden).

Anwendungstechnisch zweckmäßig umfasst die erste Reaktionsstufe des erfindungsgemäßen Verfahrens keine weiteren Reaktionszonen. D.h., das Salzbad B umströmt zweckmäßig den Abschnitt der Rohre, in welchem sich die oxidative Anschlussumsetzung des Propens (beim einfachen Durchgang) bis zu einem Umsatzwert ≥90 mol-%, oder ≥92 mol-% oder ≥94 mol% oder mehr vollzieht.

Üblicherweise liegt der Beginn der Reaktionszone B hinter dem Heißpunktmaximum der Reaktionszone A.

Die beiden Salzbäder A,B können erfindungsgemäß relativ zur Strömungsrichtung des durch die Reaktionsrohre strömenden Reaktionsgasgemisches im Gleichstrom oder im Gegenstrom durch den die Reaktionsrohre umgebenden Raum geführt werden. Selbstverständlich kann erfindungsgemäß auch in der Reaktionszone A eine Gleichströmung und in der Reaktionszone B eine Gegenströmung (oder umgekehrt) angewandt werden.

Selbstverständlich kann man in allen vorgenannten Fallkonstellationen innerhalb der jeweiligen Reaktionszone der, relativ zu den Reaktionsrohren, erfolgenden Parallelströmung der Salzschmelze noch eine Querströmung überlagern, so dass die einzelne Reaktionszone einem wie in der EP-A 700714 oder in der EP-A 700893 beschriebenen Rohrbündelreaktor entspricht und insgesamt im Längsschnitt durch das Kontaktrohr bündel ein mäanderförmiger Strömungsverlauf des Wärmeaustauschmittels resultiert.

Zweckmäßigerweise wird beim erfindungsgemäßen Verfahren das Reaktionsgasausgangsgemisch 1 der Festbettkatalysatorschüttung 1 auf die Reaktionstemperatur vorerwärmt zugeführt.

Üblicherweise sind in den Zweizonenrohrbündelreaktoren die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 21 bis 26 mm. Ihre Länge beträgt zweckmäßig 2 bis 4 m, bevorzugt 2,5 bis 3,5 m. In jeder Temperaturzone belegt die Festbettkatalysatorschüttung 1 wenigstens 60 % bzw. wenigstens 75 %, oder wenigstens 90 % der Länge der Zone. Die gegebenenfalls verbleibende Restlänge wird gegebenenfalls von einer Inertschüttung belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet (bevorzugt 6 äquidistante Nachbarrohre pro Kontaktrohr), wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt. (vgl. z.B. EP-B 468290).

Als Wärmeaustauschmittel eignen sich auch für die Zweizonenfahrweise insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweizonenrohrbündelreaktoren die Fließgeschwindigkeit innerhalb der beiden erforderlichen Wärmeaustauschmittelkreisläufen so gewählt, dass die Temperatur des Wärmeaustauschmittels von der Eintrittsstelle in die Reaktionszone bis zur Austrittstelle aus der Reaktionszone (bedingt durch die Exothermie der Reaktion) um 0 bis 15°C ansteigt. D.h., das vorgenannte ΔT kann erfindungsgemäß 1 bis 10°C, oder 2 bis 8°C oder 3 bis 6°C betragen.

Die Eintrittstemperatur des Wärmeaustauschmittels in die Reaktionszone A liegt erfindungsgemäß normalerweise im Bereich von 290 bis 380°C, bevorzugt im Bereich von 305 bis 365°C und besonders bevorzugt im Bereich von 310 bis 340°C bzw. bei 330°C. Die Eintrittstemperatur des Wärmeaustauschmittels in die Reaktionszone B liegt erfindungsgemäß normalerweise ebenfalls im Bereich von 290 bis 380°C, gleichzeitig aber normalerweise ≥0°C bis ≤20°C bzw. ≤10°C, bzw. ≥0°C und ≤5°C, oder häufig ≥0°C und ≤3°C unterhalb der Eintrittstemperatur des in die Reaktionszone A eintretenden Wärmeaustauschmittels.

Es sei an dieser Stelle auch noch einmal darauf hingewiesen, dass für eine Durchführung der Reaktionsstufe 1 des erfindungsgemäßen Verfahrens insbesondere auch der in der DE-AS 2201528 beschriebene Zweizonenrohrbündelreaktortyp verwendet werden kann, der die Möglichkeit beinhaltet, vom heißeren Wärmeaustauschmittel der Reaktionszone B eine Teilmenge an die Reaktionszone A abzuführen, um gegebenenfalls ein Anwärmen eines kalten Reaktionsgasausgangsgemisches oder eines kalten Kreisgases zu bewirken. Ferner kann die Rohrbündelcharakteristik innerhalb einer individuellen Reaktionszone wie in der EP-A 382098 beschrieben gestaltet werden.

Erfindungsgemäß hat es sich als zweckmäßig erwiesen, das die erste Reaktionsstufe verlassende Produktgasgemisch vor dem Eintritt in die zweite Reaktionsstufe auf direkte und/oder indirekte Weise abzukühlen, um so eine Nachvollverbrennung von Teilen des in der ersten Reaktionsstufe gebildeten Acroleins zu unterdrücken. Üblicherweise wird dazu zwischen die beiden Reaktionsstufen ein Nachkühler geschaltet. Dies kann im einfachsten Fall ein indirekter Rohrbündelwärmeüberträger sein. Das Produktgasgemisch wird dabei in der Regel durch die Rohre geführt und um die Rohre wird ein Wärmetauschermedium geführt, dessen Art der für die Rohrbündelreaktoren empfohlenen Wärmetauschermedien entsprechen kann. Mit Vorteil ist das Rohrinnere mit inerten Füllkörpern (z.B. Spiralen aus Edelstahl, Ringe aus Steatit, Kugeln aus Steatit etc.) gefüllt. Selbige verbessern den Wärmeaustausch und fangen gegebenenfalls aus der Festbettkatalysatorschüttung der ersten Reaktionsstufe sublimierendes Molybdäntrioxid vor einem Eintritt desselben in die zweite Reaktionsstufe ab. Es ist von Vorteil, wenn der Nachkühler aus mit Zinksilicatfarbe beschichtetem rostfreiem Stahl gefertigt ist.

In der Regel wird der auf den einfachen Durchgang bezogene Propenumsatz beim erfindungsgemäßen Verfahren in der ersten Reaktionsstufe ≥92 mol-% oder ≥94 mol-% betragen. Die in der ersten Reaktionsstufe bei einfachem Durchgang resultierende Selektivität der Acroleinbildung sowie der Acrylsäurenebenproduktbildung wird dabei erfindungsgemäß zusammen regelmäßig ≥92 mol-% oder ≥94 mol%, häufig ≥95 mol-% oder ≥96 mol% bzw. ≥97 mol-% betragen.

Als Quelle für den in der ersten Reaktionsstufe erforderlichen molekularen Sauerstoff kommt sowohl Luft, als auch an molekularem Stickstoff entreicherte Luft (z.B. ≥90 Vol.% O₂, ≤10 Vol.-% N₂) in Betracht.

Anwendungstechnisch zweckmäßig wird das Produktgasgemisch der ersten Reaktionsstufe im bereits erwähnten Nachkühler auf eine Temperatur von 210 bis 290°C, häufig 230 bis 280°C oder 250 bis 270°C abgekühlt. Dabei kann die Abkühlung des Produktgasgemisches der ersten Reaktionsstufe durchaus auf Temperaturen erfolgen, die unterhalb der Temperatur der zweiten Reaktionsstufe liegen. Die beschriebene Nachkühlung ist jedoch keineswegs zwingend und kann insbesondere dann in aller Regel entfallen, wenn der Weg des Produktgasgemisches von der ersten Reaktionsstufe in die zweite Reaktionsstufe kurz gehalten wird. Üblicherweise wird das erfindungsgemäße Verfahren ferner so verwirklicht, dass man den Sauerstoffbedarf in der zweiten Reaktionsstufe nicht bereits durch einen entsprechend hohen Sauerstoffgehalt des Reaktionsgasausgangsgemisches 1 deckt, sondern den benötigten Sauerstoff im Bereich zwischen erster und zweiter Reaktionsstufe zugibt ("Sekundärgaszusatz"). Dies kann vor, während, nach und/oder zur Nachkühlung erfolgen. Als Quelle für den in der zweiten Reaktionsstufe erforderlichen molekularen Sauerstoff kommen sowohl reiner Sauerstoff als auch Gemische aus Sauerstoff und Inertgas, z.B. Luft oder an molekularem Stickstoff entreicherte Luft (z.B. ≥90 Vol-% O₂, ≤10 Vol-% N₂) in Betracht. Die Zugabe der Sauerstoffquelle erfolgt regelmäßig in auf den Reaktionsdruck komprimierter Form. Selbstredend kann beim erfindungsgemäßen Verfahren der Sauerstoffbedarf in der zweiten Reaktionsstufe bereits durch einen entsprechend hohen Sauerstoffbedarf in der ersten Reaktionsstufe gedeckt werden. Natürlich kann bei Bedarf als Sekundärgas auch ein inertes Verdünnungsgas zugesetzt werden.

Wie die Durchführung der ersten Reaktionsstufe erfolgt auch die Durchführung der zweiten Reaktionsstufe des erfindungsgemäßen Verfahrens in anwendungstechnis ch zweckmäßiger Weise in einem Zweizonenrohrbündelreaktor, wie er für die erste Reaktionsstufe bereits beschrieben wurde. Die Ausführungen hinsichtlich des Zweizonenrohrbündelreaktors für die erste Reaktionsstufe gelten deshalb auch für den Zweizonenrohrbündelreaktor für die zweite Reaktionsstufe.

D.h., in einfacher Weise befindet sich die erfindungsgemäß zu verwendende Festbettkatalysatorschüttung 2 (gegebenenfalls einschließlich der Inertschüttungen) in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Reaktionszone.

D.h., in einfacher Weise umströmt z.B. ein Salzbad C diejenigen Abschnitte der Rohre (die Reaktionszone C), in welchem sich die oxidative Umsetzung des Acroleins (beim einfachen Durchgang) bis zum Erreichen eines Umsatzwertes im Bereich von 45 bis 85 mol-% (bevorzugt 50 bis 85 mol-%, besonders bevorzugt 60 bis 85 mol-%) vollzieht und ein Salzbad D umströmt den Abschnitt der Rohre (die Reaktionszone D), in welchem sich die oxidative Anschlussumsetzung des Acroleins (beim einfachen Durchgang) bis zum Erreichen eines Umsatzwertes von wenigstens 90 mol-% vollzieht (bei Bedarf können sich an die erfindungsgemäß anzuwendenden Reaktionszonen C, D weitere Reaktionszonen anschließen, die auf individuellen Temperaturen gehalten werden).

Anwendungstechnisch zweckmäßig umfasst die Reaktionsstufe 2 des erfindungsgemäßen Verfahrens keine weiteren Reaktionszonen. D.h., das Salzbad D umströmt zweckmäßig den Abschnitt der Rohre, in welchem sich die oxidative Anschlussumsetzung des Acroleins (beim einfachen Durchgang) bis zu einem Umsatzwert von ≥92 mol-%, oder ≥94 mol-% oder ≥96 mol-% oder ≥98 mol-% und häufig sogar ≥99 mol-% oder mehr vollzieht.

Üblicherweise liegt der Beginn der Reaktionszone D hinter dem Heißpunktmaximum der Reaktionszone C

Die beiden Salzbäder C, D können erfindungsgemäß relativ zur Strömungsrichtung des durch die Reaktionsrohre strömenden Reaktiongasgemisches im Gleichstrom oder im Gegenstrom durch den die Reaktionsrohre umgebenden Raum geführt werden. Selbstverständlich kann erfindungsgemäß auch in der Reaktionszone C eine Gleichströmung und in der Reaktionszone D eine Gegenströmung (oder umgekehrt) angewandt werden.

Selbstredend kann man in allen vorgenannten Fallkonstellationen innerhalb der jeweiligen Reaktionszone der, relativ zu den Reaktionsrohren, erfolgenden Parallelströmung der Salzschmelze noch eine Querströmung überlagern, so dass die einzelne Reaktionszone einem wie in der EP-A 700714 oder in der EP-A 700893 beschriebenen Rohrbündelreaktor entspricht und insgesamt im Längsschnitt durch das Kontaktroh r-bündel ein mäanderförmiger Strömungsverlauf des Wärmeaustauschmittels resultiert.

Üblicherweise sind in den vorgenannten Zweizonen-Rohrbündelreaktoren für die zweite Reaktionsstufe die Kontaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 22 bis 26 mm. Ihre Länge beträgt zweckmäßig 3 bis 4, bevorzugt 3,5 m. In jeder Temperaturzone belegt die Festbettkatalysatorschüttung 2 wenigstens 60 %, bzw. wenigstens 75 %, oder wenigstens 90 % der Länge der Zone. Die gegebenenfalls verbleibende Restlänge wird gegebenenfalls von einer Inertsch üttung belegt. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet (bevorzugt 6 äquidistante Nachbarrohre pro Kontaktrohr), wobei die Verteilung zweckmäßig so gewählt wird, dass der Abstand der zentrischen Innenachsen von zueinander nächstliegenden Kontaktrohren (die sogenannte Kontaktrohrteilung) 35 bis 45 mm beträgt (vgl. EP-B 468290).

Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweizonenrohrbündelreaktoren der zweiten Reaktionsstufe die Fließgeschwindigkeit innerhalb der beiden erforderlichen Wärmeaustauschmittelkreisläufe so gewählt, dass die Temperatur des Wärmeaustauschmittels von der Eintrittstelle in die Reaktionszone bis zur Austrittsstelle aus der Reaktionszone um 0 bis 15°C ansteigt. D.h., das vorgenannte ΔT kann erfindungsgemäß 1 bis 10°C, oder 2 bis 8°C oder 3 bis 6°C betragen.

Die Eintrittstemperatur des Wärmeaustauschmittels in die Reaktionszone C liegt erfindungsgemäß normalerweise im Bereich von 230 bis 320°C, bevorzugt im Bereich von 250 bis 300°C und besonders bevorzugt im Bereich von 260 bis 280°C. Die Eintrittstemperatur des Wärmeaustauschmittels in die Reaktionszone D liegt erfindungsgemäß normalerweise ebenfalls im Bereich von 230 bis 280°C, gleichzeitig aber normalerweise ≥0°C bis ≤20°C bzw. ≤10°C, bzw. ≥0°C und ≤5°C, oder häufig ≥0°C und ≤3°C unterhalb der Eintrittstemperatur des in die Reaktionszone C eintretenden Wärmeaustauschmittels.

Es sei an dieser Stelle auch noch einmal darauf hingewiesen, dass für eine Durchführung der zweiten Reaktionsstufe des erfindungsgemäßen Verfahrens insbesondere auch der in der DE-AS 2201528 beschriebene Zweizonenrohrbündelreaktortyp verwendet werden kann, der die Möglichkeit beinhaltet, vom heißeren Wärmeaustauschmittel der Reaktionszone D eine Teilmenge an die Reaktionszone C abzuführen, um gegebenenfalls ein Anwärmen eines zu kalten Reaktionsgasausgangsgemisches 2 oder eines kalten Kreisgases zu bewirken. Ferner kann die Rohrbündelcharakteristik, innerhalb einer individuellen Reaktionszone wie in der EP-A 382 098 beschrieben gestaltet werden.

Selbstredend können zur Durchführung des erfindungsgemäßen Verfahrens zwei Zweizonenrohrbündelreaktoren zu einem Vierzonenrohrbündelreaktor verschmolzen werden, wie es in der WO 01/36364 beschrieben ist. Normalerweise befindet sich in diesen Fällen zwischen der Festbettkatalysatorschüttung 1 und der Festbettkatalysatorschüttung 2 eine Inertschüttung. Allerdings kann auf eine solche Zwischeninertschüttung auch verzichtet werden. Die Länge der Reaktionsrohre entspricht im Verschmelzungsfall vielfach der Summe der Längen der nicht verschmolzenen Rohrbündelreaktoren.

Der Propenanteil im Reaktionsgasausgangsgemisch 1 kann beim erfindungsgemäßen Verfahren z.B. bei Werten von 4 bis 15 Vol.-%, häufig bei 5 bis 12 Vol.-% bzw. 5 bis 8 Vol.-% liegen (jeweils bezogen auf das Gesamtvolumen).

Häufig wird man das erfindungsgemäße Verfahren bei einem Propen : Sauerstoff : indifferente Gase (einschließlich Wasserdampf) Volumenverhältnis im Reaktionsgasausgangsgemisch 1 von 1 : (1,0 bis 3,0): (5 bis 25), vorzugsweise 1 : (1,7 bis 2,3) : (10 bis 15) durchführen. Im Regelfall wird das indifferente (inerte) Gas zu wenigstens 20 % seines Volumens aus molekularem Stickstoff bestehen. Es kann aber auch zu ≥30 Vol.-%, oder zu ≥40 Vol.-%, oder zu ≥50 Vol.-%, oder zu ≥60 Vol.-%, oder zu ≥70 Vol.-%, oder zur ≥80 Vol.-%, oder zu ≥90 Vol.-%, oder zu ≥95 Vol.-% aus molekularem Stickstoff bestehen (generell sollen in dieser Schrift inerte Verdünnungsgasse solche sein ,die sich beim einmaligen Durchgang durch die jeweilige Reaktionsstufe zu weniger als 5 %, bevorzugt zu weniger als 2 % umsetzen; das sind neben molekularem Stickstoff z.B. Gase wie Propan, Ethan, Methan, Pentan, Butan, CO₂, CO, Wasserdampf und/oder Edelgase). Natürlich kann das inerte Verdünnungsgas beim erfindungsgemäßen Verfahren auch zu bis zu 50 mol-%, bzw. bis zu 75 mol-% und mehr aus Propan bestehen. Bestandteil des Verdünnungsgases kann auch Kreisgas sein, wie es nach der Abtrennung der Acrylsäure aus dem Produktgasgemisch verbleibt.

Vorgenannte Zusammensetzungsbereiche gelten sowohl in Fällen von Sekundärgaszufuhr als auch in Fällen wo kein Sekundärgas zugeführt wird.

Erfindungsgemäß günstige Reaktionsgasausgangsgemische 1 sind z.B. auch solche, die aus

| | |
|---|---|
| 6 bis 15 (bevorzugt 7 bis 11) Vol-% | Propen, |
| 4 bis 20 (bevorzugt 6 bis 12) Vol.-% | Wasser, |
| ≥0 bis 10 (bevorzugt ≥0 bis 5) Vol.-% | von Propen, Wasser, Sauerstoff und Stickstoff verschiedenen Bestandteilen, |

soviel molekularem Sauerstoff, dass das molare Verhältnis von enthaltenem molekularem Sauerstoff zu enthaltenem Propen 1,5 bis 2,5 (bevorzugt 1,6 bis 2,2) beträgt, und als Restmenge bis zu 100 Vol.% Gesamtmenge aus molekularem Stickstoff zusammengesetzt sind,
wie sie die DE-A 10302715 empfiehlt.

Der Acroleinanteil im Reaktionsgasausgangsgemisch 2 kann erfindungsgemäß z.B. bei Werten von 3 bis 15 Vol.-%, häufig bei 4 bis 10 Vol.-% bzw. 5 bis 8 Vol.-% liegen (jeweils bezogen auf das Gesamtvolumen).

Häufig wird man das erfindungsgemäße Verfahren mit einem im Reaktionsgasausgangsgemisch 2 vorliegenden Acrolein : Sauerstoff : Wasserdampf : Inertgas - Volumenverhältnis (NI) von 1 : (1 bis 3) : (0 bis 20) : (3 bis 30), vorzugsweise von 1 : (1 bis 3) : (0,5 bis 10) : (7 bis 10) ausführen.

Selbstredend kann man das erfindungsgemäße Verfahren aber auch mit einem im Reaktionsgasausgangsgemisch 2 vorliegenden Acrolein : Sauerstoff : Wasserdampf : Sonstige - Volumenverhältnis (NI) von 1 : (0,9 bis 1,3): (2,5 bis 3,5): (10 bis 12) ausführen.

An dieser Stelle sei noch festgehalten, dass als Aktivmassen sowohl für die Festbettkatalysatorschüttung 1 als auch für die Festbettkatalysatorschüttung 2 auch die Multimetalloxidmassen der DE-A 10261186 günstig sind.

Erfindungsgemäß günstige Ausgestaltungen eines Zweizonenrohrbündelreaktors für die erste Reaktionsstufe können wie folgt beschaffen sein (die konstruktive Detailgestaltung kann wie in den Gebrauchsmusteranmeldungen 202 19 277.6, 2002 19 278.4 und 202 19 279.2 bzw. in den PCT-Anmeldungen PCT/EP02/14187, PCT/EP02/14188 oder PCT/EP02/14189 erfolgen):

### Kontaktrohre:

| | |
|---|---|
| Material der Kontaktrohre: | ferritischer Stahl; |
| Abmessungen der Kontaktrohre: | z.B. 3500 mm Länge; |
| | z.B. 30 mm Außendurchmesser; |
| | z.B. 2 mm Wandstärke; |

Anzahl der Kontaktrohre im Rohrbündel: z.B. 30000, oder 28000, oder 32000, oder 34000; zusätzlich bis zu 10 Thermorohre (wie in EP-A 873 783 und EP-A 12 70 065 beschrieben), die wie die Kontaktrohre beschickt sind (schneckenartig von ganz außen nach innen drehend) z.B. der selben Länge und Wandstärke, aber mit einem Außendurchmesser von z.B. 33,4 mm und einer zentrierten Thermohülse von z.B. 8 mm Außendurchmesser und z.B. 1 mm Wandstärke;

### Reaktor (Material wie die Kontaktrohre):

Zylinderförmiger Behälter eines Innendurchmessers von 6000 - 8000 mm;
Reaktorhauben plattiert mit Edelstahl des Typs 1,4541; Plattierungsdicke: einige mm;
ringförmig angeordnetes Rohrbündel, z.B. mit einem freien zentralen Raum;
Durchmesser des zentralen freien Raumes: z.B. 1000 - 2500 mm (z.B. 1200 mm, oder 1400 mm, oder 1600 mm, oder 1800 mm, oder 2000 mm, oder 2200 mm, oder 2400 mm);
normalerweise homogene Kontaktrohrverteilung im Rohrbündel (6 äquidistante Nachbarrohre pro Kontaktrohr), Anordnung in gleichseitigem Dreieck, Kontaktrohrteilung (Abstand der zentrischen Innenachsen von zueinander nächstliegenden Kontaktrohren); 35 - 45 mm, z.B. 36 mm, oder 38 mm, oder 40 mm, oder 42 mm, oder 44 mm ;
die Kontaktrohre sind mit ihren Enden in Kontaktrohrböden (oberer Boden und unterer Boden z.B. mit einer Dicke von 100 - 200 mm) abdichtend befestigt und münden arm oberen Ende in eine mit dem Behälter verbundene Haube, die einen Zulass für das Reaktionsgasausgangsgemisch aufweist; ein z.B. auf der halben Kontaktrohrlänge befindliches Trennblech einer Dicke von 20-100 mm, teilt den Reaktorraum symmetrisch in zwei Reaktionszonen (Temperaturzonen) A (obere Zone) und B (untere Zone); jede Reaktionszone wird durch eine Umlenkscheibe in 2 äquidistante Längsabschnitte geteilt;
die Umlenkscheibe weist bevorzugt Ringgeometrie auf; die Kontaktrohre sind mit Vorteil am Trennblech abdichtend befestigt; an den Umlenkscheiben sind sie nicht abd ichtend befestigt, so dass die Querströmungsgeschwindigkeit der Salzschmelze innerhalb einer Zone möglichst konstant ist;
jede Zone wird durch eine eigene Salzpumpe mit Salzschmelze als Wärmeträger versorgt; die Zufuhr der Salzschmelze ist z.B. unterhalb der Umlenkscheibe und die Entnahme ist z.B. oberhalb der Umlenkscheibe;
aus beiden Salzschmelzekreisen wird z.B. ein Teilstrom entnommen und z.B. in einem gemeinsamen oder zwei getrennten indirekten Wärmetauschern abgekühlt (Dampferzeugung);
im ersten Fall wird der abgekühlte Salzschmelzestrom aufgeteilt, mit dem jeweiligen Reststrom vereinigt und durch die jeweilige Pumpe in den entsprechenden Ringkanal, der die Salzschmelze über den Behälterumfang verteilt, in den Reaktor gedrückt;
durch im Reaktormantel befindliche Fenster gelangt die Salzschmelze zum Rohrbündel; die Einströmung erfolgt z.B. in radialer Richtung zum Rohrbündel;
die Salzschmelze fließt in jeder Zone der Vorgabe des Umlenkblechs folgend z.B. in der Abfolge
   - von außen nach innen,
   - von innen nach außen,
um die Kontaktrohre;
durch um den Behälterumfang angebrachte Fenster sammelt sich die Salzschmelze an jedem Zonenende in einem um den Reaktormantel angebrachten Ringkanal, um einschließlich Teilstromkühlung im Kreis gepumpt zu werden;
über jede Reaktionszone wird die Salzschmelze von unten nach oben geführt;

Das Reaktionsgasgemisch verlässt den Reaktor der ersten Stufe mit einer Temperatur wenige Grad höher als die Salzbadeintrittstemperatur des ersten Reaktors. Das Re aktionsgasgemisch wird für die Weiterverarbeitung zweckmäßigerweise in einem separaten Nachkühler, der dem Reaktor der 1. Stufe nachgeschaltet ist, auf 220°C bis 280°C, bevorzugt 240°C bis 260°C abgekühlt.

Der Nachkühler ist in der Regel unterhalb des unteren Rohrbodens angeflanscht und besteht normalerweise aus Rohren mit ferritischem Stahl. In die Rohre des Nachkühlers sind mit Vorteil innen Edelstahl-Blechspiralen, die teil- oder vollgewendelt sein können, zur Verbesserung des Wärmeübergangs eingeführt.

### Salzschmelze:

Als Salzschmelze kann ein Gemisch aus 53 Gew.-% Kaliumnitrat, 40 Gew.-% Natriumnitrit und 7 Gew.-% Natriumnitrat verwendet werden; beide Reaktionszonen und der Nachkühler wenden mit Vorteil eine Salzschmelze derselben Zusammensetzung an; die in den Reaktionszonen umgepumpte Salzmenge kann je Zone ca. 10000 m³/h betragen.

### Stromführung:

das Reaktionsgasausgangsgemisch 1 strömt zweckmäßig von oben nach unten durch den Erststufenreaktor, während die unterschiedlich temperierten Salzschmelzen der einzelnen Zonen zweckmäßig von unten nach oben gefördert werden;
Kontaktrohr- und Thermorohrbeschickung (von oben nach unten) z.B.:
   - Abschnitt 1:: 50 cm Länge Steatitringe der Geometrie 7 mm x 7 mm x 4 mm (Außendurchmesser x Länge x Innendurchmesser) als Vorschüttung.
   - Abschnitt 2:: 140 cm Länge Katalysatorbeschickung mit einem homogenen Gemisch aus 30 Gew.-% an Steatitringen der Geometrie 5 mm x 3 mm x 2 mm (Außendurchmesser x Länge x Innendurchmesser) und 70 Gew.-% Vollkatalysator aus Abschnitt 3.
   - Abschnitt 3:: 160 cm Länge Katalysatorbeschickung mit ringförmigem (5 mm x 3 mm x 2 mm = Außendurchmesser x Länge x Innendurchmesser) Vollkatalysator gemäß Beispiel 1 der DE-A 10046957 (Stöchiometrie: [Bi₂W₂O₉ x 2 WO₃]_{0,5} [Mo₁₂Co_{5,5}Fe_{2,94}Si_{1,59}K_{0,08}Oₓ]₁).

Erfindungsgemäß günstige Ausgestaltungen eines Zweizonenrohrbündelreaktors für die zweite Reaktionsstufe können wie folgt beschaffen sein:

Alles wie beim Zweizonenrohrbündelreaktor für die erste Reaktionsstufe. Die Dicke des oberen und unteren Kontaktrohrbodens beträgt jedoch häufig 100 - 200 mm, z.B. 110 mm, oder 130 mm, oder 150 mm, oder 170 mm, oder 190 mm.

Der Nachkühler entfällt; statt dessen münden die Kontaktrohre mit ihren unteren Öffnungen in eine am unteren Ende mit dem Behälter verbundene Haube mit Auslass für das Produktgasgemisch; die obere Reaktionszone ist die Zone C und die untere Reaktionszone ist die Reaktionszone D. Zwischen Auslass "Nachkühler" und Einlass "Reaktor für die zweite Reaktionsstufe" besteht zweckmäßig eine Zufuhrmöglichkeit für komprimierte Luft.

Die Kontaktrohr- und Thermorohrbeschickung (von oben nach unten) kann z.B. wie folgt sein:
- Abschnitt 1:: 20 cm Länge Steatitringe der Geometrie 7 mm x 7 mm x 4 mm (Außendurchmesser x Länge x Innendurchmesser) als Vorschüttung.
- Abschnitt 2:: 90 cm Länge Katalysatorbeschickung mit einem homogenen Gemisch aus 30 Gew.-% an Steatit-Ringen der Geometrie 7 mm x 3 mm x 4 mm (Außendurchmesser x Länge x Innendurchmesser) und 70 Gew.-% Schalenkatalysator aus Abschnitt 4.
- Abschnitt 3:: 50 cm Länge Katalysatorbeschickung mit einem homogenen Gemisch aus 20 Gew.% an Steatit-Ringen der Geometrie 7 mm x 3 mm x 4 mm (Außendurchmesser x Länge x Innendurchmesser) und 80 Gew.-% Schalenkatalsator aus Abschnitt 4.
- Abschnitt 4:: 190 cm Länge Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: Mo₁₂V₃W_{1,2}Cu_{2,4}Oₓ).

Alternativ kann die Erststufen-Kontaktrohr- und Thermorohrbeschickung (von oben nach unten) auch so aussehen:
- Abschnitt 1:: 50 cm Länge Steatitringe der Geometrie 7 mm x 7 mm x 4 mm (Außendurchmesser x Länge x Innendurchmesser) als Vorschüttung.
- Abschnitt 2:: 300 cm Länge Katalysatorbeschickung mit ringförmigem (5 mm x 3 mm x 2 mm = Außendurchmesser x Länge x Innendurchmesser) Vollkatalysator gemäß Beispiel 1 der DE-A 10046957 (Stöchiometrie: [Bi₂W₂O₉ x 2WO₃]_{0,5} • [Mo₁₂Co_{5,6}Fe_{2,94}Si_{1,59}K_{0,08}Oₓ]₁).

Die Zvsreitstufen-Kontaktrohr- und Thermorohrbeschickung kann (von oben nach unten) auch so aussehen:
- Abschnitt 1:: 20 cm Länge Steatitringe der Geometrie 7 mm x 7 mm x 4 mm (Außendurchmesser x Länge x Innendurchmesser) als Vorschüttung.
- Abschnitt 2:: 140 cm Länge Katalysatorbeschickung mit einem homogenen Gemisch aus 25 Gew.-% an Steatit-Ringen der Geometrie 7 mm x 3 mm x 4 mm (Außendurchmesser x Länge x Innendurchmesser) und 75 Gew.-% Schalenkatalysator aus Abschnitt 3.
- Abschnitt 3:: 190 cm Länge Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: MO₁₂V₃W_{1,2}Cu_{2,4}Oₓ).

In allen genannten Erststufenbeschickungen kann der Vollkatalysator aus Beispiel der DE-A 10046957 auch ersetzt werden durch:
a) einen Katalysator gemäß Beispiel 1c aus der EP-A 15565 oder einen gemäß diesem Beispiel herzustellenden Katalysator, der jedoch die Aktivmasse Mo₁₂Ni_{6,5}Zn₂Fe₂Bi₁P_{0,0065}K_{0,06}Oₓ • 10 SiO₂ aufweist;
b) Beispiel Nr. 3 aus der DE-A 19855913 als Hohlzylindervollkatalysator der Geometrie 5 mm x 3 mm x 2 mm bzw. 5 mm x 2 mm x 2 mm;
c) Multimetalloxid II - Vollkatalysator gemäß Beispie 1 der DE-A 19746210;
d) einen der Schalenkatalysatoren 1, 2 und 3 aus der DE-A 10063162, jedoch bei gleicher Schalendicke auf Trägerringe der Geometrie 5 mm x 3 mm x 1,5 mm bzw. 7 mm x 3 mm x 1,5 mm aufgebracht.

In allen vorgenannten Zweitstufenbeschickungen kann der Schalenkatalysator gemäß Herstellungsbeispiel 5 der DE-A 10046928 ersetzt werden durch:
a) Schalenkatalysator S1 oder S7 aus der DE-A 4442346 mit einem Aktivmassenanteil von 27 Gew.-% und einer Schalendicke von 230 µm;
b) einem Schalenkatalysator gemäß den Beispielen 1 bis 5 aus der DE-A 19815281, jedoch auf Trägerringe der Geometrie 7 mm x 3 mm x 4 mm mit einem Aktivmassenanteil von 20 Gew.-% aufgebracht;
c) Schalenkatalysator mit zweiphasiger Aktivmasse der Stöchiometrie (Mo_{10,4}V₃W_{1,2}Oₓ) (CuMo_{0,5}W_{0,5}O₄)_{1,6}, hergestellt gemäß DE-A 19736105 und mit einem Aktivmassenanteil von 20 Gew.-% auf den vorgenannten 7 mm x 3 mm x 4 mm Träger aufgebracht.

Im Übrigen werden die Festbettkatalysatorschüttung 1 und die Festbettkatalysatorschüttung 2 erfindungsgemäß zweckmäßig so gewählt (z.B. durch Verdünnung mit z.B. Inertmaterial), dass der Temperaturunterschied zwischen dem Heißpunktmaximum des Reaktionsgasgemisches in den einzelnen Reaktionszonen und der jeweiligen Temperatur der Reaktionszone in der Regel 80°C nicht überschreitet. Meist beträgt dieser Temperaturunterschied ≤70°C, häufig liegt er bei 20 bis 70°C, vorzugsweise ist dieser Temperaturunterschied gering. Außerdem werden die Festbettkatalysatorschüttungen 1 und 2 aus Sicherheitsgründen in dem Fachmann an sich bekannter Weise so gewählt (z.B. durch Verdünnung mit z.B. Inertmaterial), dass die "peak-to-salttemperature sensitivity" gemäß Definition in der EP-A 1106598 ≤9°C, oder ≤7°C, oder ≤5°C, oder ≤3°C beträgt.

Nachkühler und Reaktor für die zweite Stufe sind durch ein Verbindungsrohr verbunden, dessen Länge weniger als 25 m beträgt.

Die beschriebene Reaktoranordnung und alle anderen in dieser Schrift beschriebenen Reaktoranordnungen und Beschickungen mit Festbettkatalysator können auch bei hohen Propenlasten wie in den Schriften WO 01/36364, DE-A 19927624, DE-A 19948248, DE-A 19948523, DE-A 19948241, DE-A 19910506, DE-A 10302715 und EP-A 1106598 beschrieben betrieben werden.

Bevorzugt können die Reaktionszonen A, B, C, D dabei die in dieser Schrift empfohlenen Temperaturen aufweisen, jedoch so, dass die jeweils zweite Reaktionszone, gemäß der Lehre der vorgenannten Schriften, eine höhere Temperatur als die jeweils erste Reaktionszone aufweist. Die Heißpunkttemperatur in der jeweils zweiten Reaktionszone liegt vorzugsweise stets unterhalb derjenigen der jeweils ersten Reaktionszone.

Bei den erfindungsgemäßen Propenlasten resultiert jedoch mit der erfindungsgemäßen Verfahrensweise eine, relativ zu der für hohe Propenlasten empfohlenen Verfahrensweise, erhöhte Selektivität der Acrylsäurebildung.

Generell lässt sich die volumenspezifische Aktivität zwischen zwei Festbettkatalysatorschüttungszonen einer Reaktionsstufe experimentell in einfacher Weise so differenzieren, dass unter identischen Randbedingungen (vorzugsweise den Bedingungen des ins Auge gefassten Verfahrens) über Festbettkatalysatorschüttungen derselben Länge, aber jeweils der Zusammensetzung der jeweiligen Festbettkatalysatorschüttungszone entsprechend, das gleiche Propen bzw. Acrolein enthaltende Reaktionsgasausgangsgemisch geführt wird. Die höhere umgesetzte Menge an Propen bzw. Acrolein weist die höhere volumenspezifische Aktivität aus.

In den nachfolgenden Beispielen und Vergleichsbeispielen sowie in der vorstehenden Reaktoranordnung können in der zweiten Reaktionsstufe die ringförmigen Verdünnungsformkörper und die ringförmigen Katalysatorformkörper auch durch kugelförmige Verdünnungsformkörper und kugelförmige Katalysatorformkörper (jeweils mit Radius 2 bis 5 mm und mit einem Aktivmassenanteil von 10 bis 30 Gew.-%, häufig 10 bis 20 Gew.-%) ersetzt werden. Die Aussage bezüglich des Betriebs bei hohen Propenlasten gemäß den vorgenannten Schriften behält dabei ihre Gültigkeit.

### Beispiele und Vergleichsbeispiele

### a) Versuchsanordnung

Erste Reaktionsstufe:

Ein Reaktionsrohr (V2A Stahl; 30 mm Außendurchmesser, 2 mm Wandstärke, 26 mm Innendurchmesser, Länge: 350 cm, sowie ein in der Reaktionsrohrmitte zentriertes Thermorohr (4 mm Außendurchmesser) zur Aufnahme eines Thermoelements mit dem die Temperatur im Reaktionsrohr auf seiner gesamten Länge ermittelt werden kann) wird von oben nach unten wie folgt beschickt:
- Abschnitt 1:: 50 cm Länge
Steatitringe der Geometrie 7 mm x 7 mm x 4 mm (Außendurchmesser x Länge x Innendurchmesser) als Vorschüttung.
- Abschnitt 2:: 140 cm Länge
Katalysatorbeschickung mit einem homogenen Gemisch aus 30 Gew.-% an Steatitringen der Geometrie 5 mm x 3 mm x 2 mm (Außendurchmesser x Länge x Innendurchmesser) und 70 Gew.-% Vollkatalysator aus Abschnitt 3.
- Abschnitt 3:: 160 cm Länge
Katalysatorbeschickung mit ringförmigem (5 mm x 3 mm x 2 mm = Außendurchmesser x Länge x Innendurchmesser) Vollkatalysator gemäß Beispiel 1 der DE-A 10046957 (Stöchiometrie: [Bi₂W₂O₉ x 2WO₃]_{0,5} [Mo₁₂Co_{5,5}Fe_{2,94}Si_{1,59}K_{0,08}Oₓ]₁).

Von oben nach unten werden die ersten 175 cm mittels eines im Gegenstrom gepumpten Salzbades A thermostatisiert. Die zweiten 175 cm werden mittels eines im Gegenstrom gepumpten Salzbades B thermostatisiert.

### Zweite Reaktionsstufe:

Ein Reaktionsrohr (V2A Stahl; 30 mm Außendurchmesser, 2 mm Wandstärke, 26 mm Innendurchmesser, Länge: 350 cm, sowie ein in der Reaktionsrohrmitte zentriertes Thermorohr (4 mm Außendurchmesser) zur Aufnahme eines Thermoelements mit dem die Temperatur im Reaktionsrohr auf seiner gesamten Länge ermittelt werden kann) wird von oben nach unten wie folgt beschickt:
- Abschnitt 1:: 20 cm Länge
Steatitringe der Geometrie 7 mm x 7 mm x 4 mm (Außendurchmesser x Länge x Innendurchmesser) als Vorschüttung.
- Abschnitt 2:: 90 cm Länge
Katalysatorbeschickung mit einem homogenen Gemisch aus 30 Gew.-% an Steatit-Ringen der Geometrie 7 mm x 3 mm x 4 mm (Außendurchmesser x Länge x Innendurchmesser) und 70 Gew.-% Schalenkatalysator aus Abschnitt 4.
- Abschnitt 3:: 50 cm Länge
Katalysatorbeschickung mit einem homogenen Gemisch aus 20 Gew.-% an Steatit-Ringen der Geometrie 7 mm x 3 mm x 4 mm (Außendurchmesser x Länge x Innendurchmesser) und 80 Gew.-% Schalenkatalysator aus Abschnitt 4.
- Abschnitt 4:: 190 cm Länge
Katalysatorbeschickung mit ringförmigem (7 mm x 3 mm x 4 mm = Außendurchmesser x Länge x Innendurchmesser) Schalenkatalysator gemäß Herstellungsbeispiel 5 der DE-A 10046928 (Stöchiometrie: Mo₁₂V₃W_{1,2}Cu_{2,4}Oₓ).

Von oben nach untern werden die ersten 175 cm mittels eines im Gegenstrom gepumpten Salzbades C thermostatisiert. Die zweiten 175 cm werden mittels eines im Gegenstrom gepumpten Salzbades D thermostatisiert.

### Gasphasenoxidation:

Die vorstehend beschriebene erste Reaktionsstufe wird mit einem Reaktionsgasausgangsgemisch 1 der nachfolgenden Zusammensetzung kontinuierlich beschickt, wobei die Belastung und die Thermostatisierung des ersten Reaktionsrohres variiert wird:
6 bis 6,5 Vol.-% Propen,
3 bis 3,5 Vol.-% H₂O,
0,3 bis 0,5 Vol.-% CO,
0,8 bis 1,2 Vol.% CO₂,
0,01 bis 0,04 Vol.-% Acrolein,
10,4 bis 10,7 Vol.-% O₂ und
als Restmenge ad 100 % molekularer Stickstoff.

Dem Produktgasgemisch der ersten Reaktionsstufe wird am Ausgang des ersten Reaktionsrohres eine kleine Probe für eine gaschromatographische Analyse entnommen. In übrigen wird das Produktgasgemisch unter Zudüsen von eine Temperatur von 25°C aufweisender Luft unmittelbar in die nachfolgende Acroleinoxidationsstufe (zu Acrylsäure) geführt (Reaktionsstufe 2).

Von Produktgasgemisch der Acroleinoxidationsstufe wird ebenfalls eine kleine Probe für eine gaschromatographische Analyse entnommen. Im übrigen wird die Acrylsäure von Produktgasgemisch der zweiten Reaktionsstufe in an sich bekannter Weise abgetrennt und ein Teil des verbleibenden Restgases zur Beschickung der Propenoxidationsstufe wiederverwendet (als sogenanntes Kreisgas), was den Acroleingehalt des vorgenannten Beschickungsgases und die geringe Varianz der Feedzusammensetzung erklärt.

In beiden Reaktionsstufen durchströmt das Reaktionsgasgemisch die beiden Kontaktrohre von oben nach unten.

Der Druck am Eingang der ersten Reaktionsstufe variiert in Abhängigkeit von der gewählten Propenbelastung zwischen 2,3 und 3,1 bar. Am Ende der Reaktionszonen A, C befindet sich ebenfalls eine Analysenstelle. Der Druck am Eingang der zweiten Reaktionsstufe variiert in Abhängigkeit von der Acroleinbelastung zwischen 1,6 und 2,1 bar.

Die Ergebnisse in Abhängigkeit von Belastungen und Salzbadtemperaturen sowie Luftzugabe nach der ersten Reaktionsstufe zeigt die nachfolgende Tabelle (der Buchstabe B in Klammern bedeutet Beispiel und der Buchstabe V in Klammern bedeutet Vergleichsbeispiel).
- T_{A}, T_{B}, T_{C}, T_{D}: stehen für die Temperaturen der umgepumpten Salzbäder in den Reaktionszonen A, B, C und D in °C.
- U_{PA}: ist der Propenumsatz am Ende der Reaktionszone A in mol-%.
- U_{PB}: ist der Propenumsatz am Ende der Reaktionszone B in mol-%.
- S_{WP}: ist die Selektivität der Acroleinbildung sowie der Acrylsäurenebenproduktbildung zusammengenommen nach der ersten Reaktionsstufe und bezogen auf umgesetztes Propen in mol-%.
- U_{AC}: ist der Acroleinumsatz am Ende der Reaktionszone C in mol-%.
- U_{AD}: ist der Acroleinumsatz am Ende der Reaktionszone D in mol-%.
- U_{PD}: ist der Propenumsatz am Ende der Reaktionszone D in mol-% (bezogen auf die Eingangsmenge an Propen in die Reaktionszone A).
- S^{AA}: ist die Selektivität der Acrylsäurebildung nach der zweiten Reaktionsstufe und bezogen auf umgesetztes Propen in mol-%.
- V: ist das molare Verhältnis von molekularem Sauerstoff : Acrolein im Reaktionsgasausgangsgemisch 2
- M: ist die Luftzugabe nach der ersten Reaktionsstufe in Nl/h.
- T^{maxA}, T^{maxB}, T^{maxC}, T^{maxD}: stehen für die höchste Temperatur des Reaktionsgasgemisches innerhalb der Reaktionszonen A, B, C und D in °C.

### b) Ergebnisse

**Tabelle**

| Propenbelastung (NI/I•h) | T_{A} | T_{B} | T^{maxA} | T^{maxB} | U_{PA} | U_{PB} | S_{WP} | M | V |
|---|---|---|---|---|---|---|---|---|---|
| 130 (B) | 319 | 319 | 384 | 351 | 66,7 | 95,1 | 97,7 | 404 | 1,40 |
| 130 (B) | 327 | 313 | 400 | 330 | 70,3 | 94,8 | 98,3 | 404 | 1,38 |
| 130 (V) | 311 | 325 | 361 | 372 | 60,8 | 95,0 | 97,1 | 404 | 1,41 |
| 185 (V) | 322 | 336 | 380 | 368 | 64,5 | 94,9 | 98,0 | 574 | 1,39 |
| 185 (V) | 310 | 344 | 353 | 383 | 57,2 | 95,2 | 97,3 | 574 | 1,40 |

**Tabelle Fortsetzung**

| Acroleinbelastung (Nl/l•h) | T_{c} | T_{D} | T^{maxC} | T^{maxD} | U_{AC} | U_{AD} | U_{PD} | S^{AA} |
|---|---|---|---|---|---|---|---|---|
| 106(B) | 260 | 260 | 302 | 276 | 80,7 | 99,3 | 95,1 | 95,4 |
| 108 (B) | 262 | 259 | 312 | 275 | 84,8 | 99,3 | 94,8 | 95,8 |
| 104 (V) | 257 | 262 | 285 | 291 | 64,0 | 99,3 | 95,0 | 94,9 |
| 152 (V) | 263 | 269 | 303 | 287 | 78,8 | 99,3 | 94,9 | 95,8 |
| 147 (V) | 257 | 278 | 278 | 310 | 61,3 | 99,3 | 95,2 | 95,0 |

## Patentansprüche

1. Verfahren der heterogen katalysierten partiellen Gasphasenoxidation von Propen zu Acrylsäure, bei dem man ein Propen, molekularen Sauerstoff und wenigstens ein Inertgas enthaltendes Reaktionsgasausgangsgemisch 1, das den molekularen Sauerstoff und das Propen in einem molaren Verhältnis O₂ : C₃H₆ ≥1 ent hält, in einer ersten Reaktionsstufe so über eine Festbettkatalysatorschüttung 1, die in zwei räumlich aufeinanderfolgenden Reaktionszonen A, B angeordnet ist, wobei die Temperatur der Reaktionszone A eine Temperatur im Bereich von 290 bis 380°C und die Temperatur der Reaktionszone B ebenfalls eine Temperatur in Bereich von 290 bis 380°C ist, und deren Aktivmasse wenigstens ein die Elemente Mo, Fe und Bi enthaltendes Multimetalloxid ist, führt, dass sich die Reaktionszone A bis zu einem Umsatz des Propens von 40 bis 80 mol.-% erstreckt und der Propenumsatz bei einmaligem Durchgang durch die Festbettkatalysatorschüttung 1 ≥90 mol.-% und die damit einhergehende Selektivität der Acroleinbildung sowie der Acrylsäurenebenproduktbildung zusammengenommen ≥90 mol.-% betragen, die Temperatur des die erste Reaktionsstufe verlassenden Produktgasgemisches durch Kühlung gegebenenfalls verringert und dem Produktgasgemisch gegebenenfalls molekularen Sauerstoff und/oder Inertgas zugibt, und danach das Produktgasgemisch als Acrolein, molekularen Sauerstoff und wenigstens ein Inertgas enthaltendes Reaktionsgasausgangsgemisch 2, das den molekularen Sauerstoff und das Acrolein in einem molaren Verhältnis O₂ : C₃H₄O ≥0,5 enthält, in einer zweiten Reaktionsstufe so über eine Festbettkatalysatorschüttung 2, die in zwei räumlich aufeinanderfolgenden Reaktionszonen C, D angeordnet ist, wobei die Temperatur der Reaktionszone C eine Temperatur im Bereich 230 bis 320°C und die Temperatur der Reaktionszone D ebenfalls eine Temperatur im Bereich 230 bis 320°C ist, und deren Aktivmasse wenigstens ein die Elemente Mo und V enthaltendes Multimetalloxid ist, führt, dass sich die Reaktionszone C bis zu einem Umsatz des Acroleins von 45 bis 85 mol.-% erstreckt und der Acroleinumsatz bei einmaligem Durchgang durch die Festbettkatalysatorschüttung 2 ≥90 mol.-% und die Selektivität der über alle Reaktionszonen bilanzierten Acrylsäurebildung, bezogen auf umgesetztes Propen, ≥80 mol.% beträgt, wobei die zeitliche Abfolge, in der das Reaktionsgasgemisch die Reaktionszonen durchströmt der alphabetischen Abfolge der Reaktionszonen entspricht, **dadurch gekennzeichnet, dass**
a) die Belastung der Festbettkatalysatorschüttung 1 mit dem im Reaktionsgasausgangsgemisch 1 enthaltenen Propen < 160 Nl Propen / I Festbettkatalysatorschüttung 1 • h und ≥90 Nl Propen / I Festbettkatalysatorschüttung 1 • h beträgt;
b) die volumenspezifische Aktivität der Festbettkatalysatorschüttung 1 in Strömungsrichtung des Reaktionsgasgemisches über die Festbettkatalysatorschüttung 1 entweder konstant ist oder wenigstens einmal zunimmt;
c) die Differenz T^{maxA} - T^{maxB}, gebildet aus der höchsten Temperatur T^{maxA}, die das Reaktionsgasgemisch innerhalb der Reaktionszone A aufweist, und der höchsten Temperatur T^{maxB}, die das Reaktionsgasgemisch innerhalb der Reaktionszone B aufweist, ≥0°C beträgt;
d) die Belastung der Festbettkatalysatorschüttung 2 mit dem im Reaktionsgasausgangsgemisch 2 enthaltenen Acrolein ≤145 NI Acrolein / Festbettkatalysatorschüttung 2 • h und ≥ 70 NI Acrolein / I Festbettkatalysatorschüttung 2 • h beträgt;
e) die volumenspezifische Aktivität der Festbettkatalysatorschüttung 2 in Strömungsrichtung des Reaktionsgasgemisches über die Festbettkatalysatorschüttung 2 wenigstens einmal zunimmt; und
f) die Differenz T^{maxC} - T^{maxD}, gebildet aus der höchsten Temperatur T^{maxC}, die das Reaktionsgasgemisch innerhalb der Reaktionszone C aufweist, und der höchsten Temperatur T^{maxD}, die das Reaktionsgasgemisch innerhalb der Reaktionszone D aufweist, ≥0°C beträgt.

2. Verfahren nach Anspruch 1, **dadurch gekennzeichnet, dass** die Differenz T^{maxA} - T^{maxB} ≥3°C und ≤70°C beträgt.

3. Verfahren nach Anspruch 1, **dadurch gekennzeichnet, dass** die Differenz T^{maxA} - T^{maxB} ≥20°C und ≤60°C beträgt.

4. Verfahren nach einem der Ansprüche 1 bis 3, **dadurch gekennzeichnet, dass** die Differenz T^{maxC}- T^{maxD} ≥3°C und ≤60°C beträgt.

5. Verfahren nach einem der Ansprüche 1 bis 3, **dadurch gekennzeichnet, dass** die Differenz T^{maxC}- T^{maxD} ≥5°C und ≤40°C beträgt.

6. Verfahren nach einem der Ansprüche 1 bis 5, **dadurch gekennzeichnet, dass** die Belastung der Festbettkatalysatorschüttung 1 mit dem im Reaktionsgasausgangsgemisch enthaltenen Propen ≥100 NI Propen/l • h und ≤150 NI Propen/l • h beträgt.

7. Verfahren nach einem der Ansprüche 1 bis 5, **dadurch gekennzeichnet, dass** die Belastung der Festbettkatalysatorschüttung 1 mit dem im Reaktionsgasausgangsgemisch enthaltenen Propen ≥110 NI Propen/l • h und ≤145 NI Propen/l • h beträgt.

8. Verfahren nach einem der Ansprüche 1 bis 7, **dadurch gekennzeichnet, dass** die Differenz T_{B} - T_{A} zwischen der Temperatur der Reaktionszone B, T_{B}, und der Temperatur der Reaktionszone A, T_{A}, ≥-10°C und ≤0°C beträgt.

9. Verfahren nach einem der Ansprüche 1 bis 8, **dadurch gekennzeichnet, dass** die Differenz T_{C}-T_{D} zwischen der Temperatur der Reaktionszone D, T_{D}, und der Temperatur der Reaktionszone C, T_{C}, ≥-10°C und ≤50°C beträgt.

10. Verfahren nach einem der Ansprüche 1 bis 9, **dadurch gekennzeichnet, dass** die Temperatur der Reaktionszone A 305 bis 365°C beträgt.

11. Verfahren nach einem der Ansprüche 1 bis 9, **dadurch gekennzeichnet, dass** die Temperatur der Reaktionszone A 310 bis 340°C beträgt.

12. Verfahren nach einem der Ansprüche 1 bis 11, **dadurch gekennzeichnet, dass** die Temperatur der Reaktionszone C 250 bis 300°C beträgt.

13. Verfahren nach einem der Ansprüche 1 bis 12, **dadurch gekennzeichnet, dass** die Temperatur der Reaktionszone C 260 bis 280°C beträgt.

14. Verfahren nach einem der Ansprüche 1 bis 13, **dadurch gekennzeichnet, dass** die Aktivmasse der Festbettkatalysatorschüttung 1 wenigstens eine Multimetalloxidaktivmasse der allgemeinen Formel I
Mo₁₂BiₐFe_{b}X¹_{c}X²_{d}X³ₑX⁴_{f}Oₙ (I),
mit
X¹ = Nickel und/oder Kobalt,
X² = Thallium, ein Alkalimetall und/oder ein Erdalkalimetall,
X³ = Zink, Phosphor, Arsen, Bor, Antimon, Zinn, Cer, Blei und/oder Wolfram,
X⁴ = Silicium, Aluminium, Titan und/oder Zirkonium,
a = 0,5 bis 5,
b = 0,01 bis 5,
c = 0 bis 10,
d = 0 bis 2,
e = 0 bis 8,
f = 0 bis 10 und
n = eine Zahl, die durch die Wertigkeit und Häufigkeit der von Sauerstoff verschiedenen Elemente in I bestimmt wird,
ist.

15. Verfahren nach einem der Ansprüche 1 bis 14, **dadurch gekennzeichnet, dass** die Aktivmasse der Festbettkatalysatorschüttung 2 wenigstens eine Multimetalloxidaktivmasse der allgemeinen Formel IV
MO₁₂VₐX¹_{b}X²_{c}X³_{d}X⁴ₑX⁵_{f}X⁶_{g}Oₙ (IV),
in der die Variablen folgende Bedeutung haben:
X¹ = W, Nb, Ta, Cr und/oder Ce,
X² = Cu, Ni, Co, Fe, Mn und/oder Zn,
X³ = Sb und/oder Bi,
X⁴ = eines oder mehrere Alkalimetalle,
X⁵ = eines oder mehrere Erdalkalimetalle,
X⁶ = Si, Al, Ti und/oder Zr,
a = 1 bis 6,
b = 0,2 bis 4,
c = 0,5 bis 18,
d = 0 bis 40,
e = 0 bis 2,
f = 0 bis 4,
g = 0 bis 40 und
n = eine Zahl, die durch die Wertigkeit und Häufigkeit der von Sauerstoff verschiedenen Elemente in IV bestimmt wird,
ist.

## Claims

1. A process for partially oxidizing propene to acrylic acid in the gas phase under heterogeneous catalysis by conducting a starting reaction gas mixture 1 which comprises propene, molecular oxygen and at least one inert gas and contains the molecular oxygen and the propene in a molar O₂ : C₃H₆ ratio of ≥ 1 in a first reaction stage over a fixed catalyst bed 1 which is arranged in two spatially successive reaction zones A, B, the temperature of reaction zone A being a temperature in the range from 290 to 380°C and the temperature of reaction zone B likewise being a temperature in the range from 290 to 380°C, and whose active composition is at least one multimetal oxide comprising the elements Mo, Fe and Bi, in such a way that reaction zone A extends to a propene conversion of from 40 to 80 mol% and the propene conversion on single pass through the fixed catalyst bed 1 is ≥ 90 mol% and the accompanying selectivity of acrolein formation and also of acrylic acid by-production taken together is ≥ 90 mol%, the temperature of the product gas mixture leaving the first reaction stage is optionally reduced by cooling and molecular oxygen and/or inert gas are optionally added to the product gas mixture, and then the product gas mixture, as a starting reaction gas mixture 2 comprising acrolein, molecular oxygen and at least one inert gas and containing the molecular oxygen and the acrolein in a molar O₂ : C₃H₄O ratio of ≥ 0.5, is conducted in a second reaction stage over a fixed catalyst bed 2 which is arranged in two spatially successive reaction zones C, D, the temperature of reaction zone C being a temperature in the range from 230 to 320°C and the temperature of reaction zone D likewise being a temperature in the range from 230 to 320°C, and whose active composition is at least one multimetal oxide comprising the elements Mo and V, in such a way that reaction zone C extends to an acrolein conversion of from 45 to 85 mol% and the acrolein conversion on single pass through the fixed catalyst bed 2 is ≥ 90 mol% and the selectivity of acrylic acid formation assessed over all reaction zones, based on converted propene, is ≥ 80 mol%, the sequence in time in which the reaction gas mixture flows through the reaction zones corresponding to the alphabetic sequence of the reaction zones, wherein
a) the hourly space velocity of the propene contained in the starting reaction gas mixture 1 on the fixed catalyst bed 1 is ≤ 160 1 (STP) of propene/l of fixed catalyst bed 1 • h and ≥ 90 1 (STP) of propene/l of fixed catalyst bed 1 • h;
b) the volume-specific activity of the fixed catalyst bed 1 is either constant or increases at least once in the flow direction of the reaction gas mixture over the fixed catalyst bed 1;
c) the difference T^{maxA} - T^{maxB}, formed from the highest temperature T^{maxA} which the reaction gas mixture has within reaction zone A and the highest temperature T^{maxB} which the reaction gas mixture has within reaction zone B, is ≥ 0°C;
d) the hourly space velocity of the acrolein contained in the starting reaction gas mixture 2 on the fixed catalyst bed 2 is < 145 1 (STP) of acrolein/l of fixed catalyst bed 2 • h and ≥ 70 1 (STP) of acrolein/l of fixed catalyst bed 2 • h;
e) the volume-specific activity of the fixed catalyst bed 2 increases at least once in the flow direction of the reaction gas mixture over the fixed catalyst bed 2; and
f) the difference T^{maxC} - T^{maxD}, formed from the highest temperature T^{maxC} which the reaction gas mixture has within reaction zone C and the highest temperature T^{maxD} which the reaction gas mixture has within reaction zone D, is ≥ 0°C.

2. A process as claimed in claim 1, wherein the difference T^{maxA} - T^{maxB} is ≥ 3°C and ≤ 70°C.

3. A process as claimed in claim 1, wherein the difference T^{maxA} - T^{maxB} is ≥ 20°C and ≤ 60°C.

4. A process as claimed in any of claims 1 to 3, wherein the difference T^{maxC} - T^{maxD} is ≥ 3°C and ≤ 60°C.

5. A process as claimed in any of claims 1 to 3, wherein the difference T^{maxc_} T^{maxD} is ≥ 5°C and ≤ 40°C.

6. A process as claimed in any of claims 1 to 5, wherein the hourly space velocity of the propene contained in the starting reaction gas mixture on the fixed catalyst bed 1 is ≥ 100 1 (STP) of propene/l • h and ≤ 150 1 (STP) of propene/l • h.

7. A process as claimed in any of claims 1 to 5, wherein the hourly space velocity of the propene contained in the starting reaction gas mixture on the fixed catalyst bed 1 is ≥ 110 1 (STP) of propene/l • h and ≤ 145 1 (STP) of propene/l • h.

8. A process as claimed in any of claims 1 to 7, wherein the difference T_{B} - T_{A} between the temperature of reaction zone B, T_{B}, and the temperature of reaction zone A, T_{A}, is ≥ -10°C and ≤ 0°C.

9. A process as claimed in any of claims 1 to 8, wherein the difference T_{C}-T_{D} between the temperature of reaction zone D, T_{D}, and the temperature of reaction zone C, T_{c}, is ≥ -10°C and ≤ 0°C.

10. A process as claimed in any of claims 1 to 9, wherein the temperature of reaction zone A is from 305 to 365°C_

11. A process as claimed in any of claims 1 to 9, wherein the temperature of reaction zone A is from 310 to 340°C.

12. A process as claimed in any of claims 1 to 11, wherein the temperature of reaction zone C is from 250 to 300°C.

13. A process as claimed in any of claims 1 to 12, wherein the temperature of reaction zone C is from 260 to 280°C.

14. A process as claimed in any of claims 1 to 13, wherein the active composition of the fixed catalyst bed 1 is at least one multimetal oxide active composition of the general formula I
Mo₁₂BiₐFe_{b}X¹_{c}X²_{d}X³ₑX⁴_{f}Oₙ (I)
where
X¹ =nickel and/or cobalt,
X² =thallium, an alkali metal and/or an alkaline earth metal,
X³ =zinc, phosphorus, arsenic, boron, antimony, tin, cerium, lead and/or tungsten,
X⁴ = silicon, aluminum, titanium and/or zirconium,
a =from 0.5 to 5,
b =from 0.01 to 5,
c =from 0 to 10,
d =from 0 to 2,
e =from 0 to 8,
f =from 0 to 10 and
n =a number which is determined by the valency and frequency of the elements other than oxygen in I.

15. A process as claimed in any of claims 1 to 14, wherein the active composition of the fixed catalyst bed 2 is at least one multimetal oxide active composition of the general formula IV
Mo₁₂VₐX¹_{b}X²_{c}X³_{d}X⁴ₑX⁵_{f}X⁶_{g}Oₙ (IV)
where the variables are defined as follows:
X¹ =W, Nb, Ta, Cr and/or Ce,
X² = Cu, Ni, Co, Fe, Mn and/or Zn,
X³ =Sb and/or Bi,
X⁴ = one or more alkali metals,
X⁵ =one or more alkaline earth metals,
X⁶ =Si, Al, Ti and/or Zr,
a =from 1 to 6,
b =from 0.2 to 4,
c =from 0.5 to 18,
d =from 0 to 40,
e =from 0 to 2,
f =from 0 to 4,
g =from 0 to 40 and
n =a number which is determined by the valency and frequency of the elements other than oxygen in IV.

## Revendications

1. Procédé d'oxydation partielle en phase gazeuse catalyséé de manière hétérogène de propène pour donner de l'acide acrylique, dans lequel un mélange initial 1 de gaz réactionnel contenant du propène, de l'oxygène moléculaire et au moins un gaz inerte, qui contient l'oxygène moléculaire et le propène en un rapport molaire O₂:C₃H₆ ≥ 1, est amené dans une première étape réactionnelle sur un remplissage 1 de catalyseur à lit fixe, agencé dans deux zones réactionnelles A, B se suivant spatialement, la température de la zone réactionnelle A étant dans la plage de 290 à 380°C et la température de la zone réactionnelle B étant également dans la plage de 290 à 380°C, et la masse active de ce remplissage étant au moins un oxyde multimétallique contenant les éléments Mo, Fe et Bi, de sorte que la zone réactionnelle A s'étend jusqu'à une conversion du propène de 40 à 80 % en moles et la conversion de propène lors d'un passage unique à travers le remplissage 1 de catalyseur à lit fixe est ≥ 90 % en moles et la sélectivité décroissante de la formation d'acroléine ainsi que la production de sous-produits d'acide acrylique est, prise dans son ensemble, ≥ 90 % en moles, puis la température du mélange gazeux de production quittant la première étape réactionnelle est éventuellement diminuée par refroidissement et de l'oxygène moléculaire et/ou du gaz inerte est éventuellement ajouté au mélange gazeux de production, et le mélange gazeux de production est amené après cela en tant que mélange initial 2 de gaz réactionnel contenant de l'acroléine, de l'oxygène moléculaire et au moins un gaz inerte, qui contient l'oxygène moléculaire et l'acroléine en un rapport molaire O₂:C₃H₄O ≥ 0,5, dans une deuxième étape réactionnelle sur un remplissage 2 de catalyseur à lit fixe, agencé dans deux zones réactionnelles C, D se suivant spatialement, la température de la zone réactionnelle C étant dans la plage de 230 à 320°C et la température de la zone réactionnelle D étant également dans la plage de 230 à 320°C, et la masse active de ce remplissage étant au moins un oxyde multimétallique contenant les éléments Mo et V, de sorte que la zone réactionnelle C s'étend jusqu'à une conversion de l'acroléine de 45 à 85 % en moles et la conversion de l'acroléine lors d'un passage unique à travers le remplissage 2 de catalyseur à lit fixe est ≥ 90 % en moles et la sélectivité de la formation d'acide acrylique équilibrée sur toutes les zones réactionnelles, par rapport au propène réagi, est ≥ 80 % en moles, la séquence temporelle selon laquelle le mélange de gaz réactionnel parcourt les zones réactionnelles correspondant à la séquence alphabétique des zones réactionnelles, **caractérisé en ce que** :
a) la charge du remplissage 1 de catalyseur à lit fixe avec le propène contenu dans le mélange initial 1 de gaz réactionnel est < 160 Nl de propène/l de remplissage 1 de catalyseur à lit fixe-h et ≥ 90 N1 de propène/l de remplissage 1 de catalyseur à lit fixe·h ;
b) l'activité volumique spécifique du remplissage 1 de catalyseur à lit fixe dans le sens du courant du mélange de gaz réactionnel sur le remplissage 1 de catalyseur à lit fixe est constante ou augmente au moins une fois;
c) la différence T^{maxA} - T^{maxB}, formée à partir de la température T^{maxA} la plus élevée que présente le mélange de gaz réactionnel à l'intérieur de la zone réactionnelle A et de la température T^{maxB} la plus élevée que présente le mélange de gaz réactionnel à l'intérieur de la zone réactionnelle B, est ≥ 0°C ;
d) la charge du remplissage 2 de catalyseur à lit fixe avec l'acroléine contenue dans le mélange initial 2 de gaz réactionnel est ≤ 145 Nl d'acroléine/l de remplissage 2 de catalyseur à lit fixe-h et ≥ 70 NI d'acroléine/l de remplissage 2 de catalyseur à lit fixe·h ;
e) l'activité volumique spécifique du remplissage 2 de catalyseur à lit fixe dans le sens du courant du mélange de gaz réactionnel sur le remplissage 2 de catalyseur à lit fixe augmente au moins une fois ; et
f) la différence T^{maxC} - T^{maxD}, formée à partir de la température T^{maxC} la plus élevée que présente le mélange de gaz réactionnel à l'intérieur de la zone réactionnelle C, et de la température T^{maxD} la plus élevée que présente le mélange de gaz réactionnel à l'intérieur de la zone réactionnelle D, est ≥ 0°C.

2. Procédé selon la revendication 1, **caractérisé en ce que** la différence T^{maxA} - T^{maxB} est ≥ 3°C et ≤ 70°C.

3. Procédé selon la revendication 1, **caractérisé en ce que** la différence T^{maxA} - T^{maxB} est ≥ 20°C et ≤ 60°C.

4. Procédé selon l'une quelconque des revendications 1 à 3, **caractérisé en ce que** la différence T^{maxC} -T^{maxD} est ≥ 3°C et ≤ 60°C.

5. Procédé selon l'une quelconque des revendications 1 à 3, **caractérisé en ce que** la différence T^{maxC} -T^{maxD} est ≥ 5°C et ≤ 40°C.

6. Procédé selon l'une quelconque des revendications 1 à 5, **caractérisé en ce que** la charge du remplissage 1 de catalyseur à lit fixe avec le propène contenu dans le mélange initial de gaz réactionnel est ≥ 100 NI de propène/1-h et ≤ 150 NI de propène/l·h.

7. Procédé selon l'une quelconque des revendications 1 à 5, **caractérisé en ce que** la charge du remplissage 1 de catalyseur à lit fixe avec le propène contenu dans le mélange initial de gaz réactionnel est ≥ 100 N1 de propène/1·h et ≤ 145 N1 de propène/l·h.

8. Procédé selon l'une quelconque des revendications 1 à 7, **caractérisé en ce que** la différence T_{B} - T_{A} entre la température T_{B} de la zone réactionnelle B et la température T_{A} de la zone réactionnelle A est ≥ -10°C et ≤ 0°C.

9. Procédé selon l'une quelconque des revendications 1 à 8, **caractérisé en ce que** la différence T_{c} - T_{D} entre la température T_{D} de la zone réactionnelle D et la température T_{c} de la zone réactionnelle C est ≥ -10°C et ≤ 0°C.

10. Procédé selon l'une quelconque des revendications 1 à 9, **caractérisé en ce que** la température de la zone réactionnelle A est de 305 à 365°C.

11. Procédé selon l'une quelconque des revendications 1 à 9, **caractérisé en ce que** la température de la zone réactionnelle A est de 310 à 340°C.

12. Procédé selon l'une quelconque des revendications 1 à 11, **caractérisé en ce que** la température de la zone réactionnelle C est de 250 à 300°C.

13. Procédé selon l'une quelconque des revendications 1 à 12, **caractérisé en ce que** la température de la zone réactionnelle C est de 260 à 280°C.

14. Procédé selon l'une quelconque des revendications 1 à 13, **caractérisé en ce que** la masse active du remplissage 1 de catalyseur à lit fixe est constituée d'au moins une masse active d'oxyde multimétallique de formule générale I:
Mo₁₂BiₐFe_{b}X¹_{c}X²_{d}X³ₑX⁴_{f}Oₙ (I),
avec
X¹ = nickel et/ou cobalt,
X² = thallium, un métal alcalin et/ou un métal alcalino-terreux,
X³ =zinc, phosphore, arsenic, bore, antimoine, étain, cérium, plomb et/ou tungstène,
X⁴ = silicium, aluminium, titane et/ou zirconium,
a = 0,5 à 5,
b = 0,01 à 5,
c = 0 à 10,
d = 0 à 2,
e = 0 à 8,
f = 0 à 10 et
n = un nombre qui est déterminé grâce à la valence et à la fréquence des éléments différents de l'oxygène dans I.

15. Procédé selon l'une quelconque des revendications 1 à 14, **caractérisé en ce que** la masse active du remplissage 2 de catalyseur à lit fixe est constituée d'au moins une masse active d'oxyde multimétallique de formule générale IV :
Mo₁₂VₐX¹_{b}X²_{c}X³_{d}X⁴ₑX⁵_{f}X⁶_{g}Oₙ (IV),
dans laquelle les variables ont la signification suivante :
X¹ = W, Nb, Ta, Cr et/ou Ce,
X² = Cu, Ni, Co, Fe, Mn et/ou Zn,
X³ = Sb et/ou Bi,
X⁴ = un ou plusieurs métaux alcalins,
X⁵ = un ou plusieurs métaux alcalino-terreux,
X⁶ = Si, Al, Ti et/ou Zr,
a = 1 à 6,
b = 0,2 à 4,
c = 0,5 à 18,
d = 0 à 40,
e = 0 à 2,
f = 0 à 4,
g = 0 à 40 et
n = un nombre qui est déterminé grâce à la valence et à la fréquence des éléments différents de l'oxygène dans IV.
